# EUROPEAN PATENT APPLICATION

(11) **EP 4 151 083 A1**
(43) Date of publication of application: **22.03.2023**
(21) Application number: 21805155.5
(22) Date of filing: 11.05.2021
(51) Int. Cl.: A01H 5/00, A01H 4/00, A01H 5/02, A01H 5/06, A01H 5/10, A01H 5/12, A23L 27/00, A61K 8/60, A61K 8/9789, A61K 36/28, A61P 43/00, A61Q 13/00, C12N 5/04, C12N 15/29, C12P 19/56, C12Q 1/68

(54) **STEVIA PLANT WITH HIGH CONTENT OF REBAUDIOSIDE E**

(30) Priority: 12.05.2020 JP 2020084130
(71) Applicant: Suntory Holdings Limited, Osaka 530-8203 (JP)
(72) Inventor: HIRAI, Tadayoshi, Soraku-gun, Kyoto 619-0284 (JP); IWAKI, Kazunari, Kawasaki-shi, Kanagawa 211-0067 (JP); OCHIAI, Kentaro, Soraku-gun, Kyoto 619-0284 (JP); TAKEYAMA, Saori, Kawasaki-shi, Kanagawa 211-0067 (JP); MIYAGAWA, Katsuro, Soraku-gun, Kyoto 619-0284 (JP)
(74) Representative: Mewburn Ellis LLP
(86) International application number: PCT/JP2021/017955
(87) International publication number: WO 2021/230258

(57) **Abstract**

Provided is a plant having at least one of genetic characteristics (A)-(E) below. (A) Alleles in which the base at the position corresponding to position 40 of SEQ ID: 1 is C are homozygous or heterozygous. (B) Alleles in which the base at the position corresponding to position 21 of SEQ ID:2 is T are homozygous or heterozygous. (C) Alleles in which the base at the position corresponding to position 28 of SEQ ID:3 is T are homozygous or heterozygous. (D) Alleles in which the base at the position corresponding to position 59 of SEQ ID:4 is C are homozygous or heterozygous. (E) Alleles in which the base at the position corresponding to position 64 of SEQ ID:5 is T are homozygous or heterozygous.

## Description

### TECHNICAL FIELD

The present invention relates to a stevia plant with high content of rebaudioside E, a screening method thereof, etc.

### BACKGROUND ART

In response to consumers' diversified needs, various drinks have been developed and are commercially available. Saccharides such as sucrose are components very commonly blended in drinks for the purpose of, for example, conferring sweetness. However, their influence on health due to excessive consumption has been pointed out. Thus, there are growing needs for lower calorie and naturally derived sweeteners. For example, Patent Literature 1 discloses a functional sweetener composition containing a vitamin, a high intensity sweetener, and a sweetness improving composition.

Steviol glycoside is known as a sweet component contained in a stevia extract. The stevia extract is mainly extracted and purified from a stevia leaf. Stevia is a perennial plant of the family *Asteraceae* with Paraguay in the South America as its place of origin, and its scientific name is *Stevia rebaudiana* Bertoni. Stevia contains a component having approximately 300 or more times the sweetness of sugar and is therefore cultivated for use of this sweet component extracted therefrom as a natural sweetener. The presence of various glycosides such as rebaudioside A (hereinafter, "rebaudioside A" is also abbreviated as "Reb"), RebB, RebC, RebD, RebE and RebD has been reported as steviol glycoside (Patent Literature 2). Among various steviol glycosides, for example, RebA is evaluated as a high intensity sweetener having good quality of sweetness and is widely used. The other steviol glycosides have also been increasingly found to have their unique sweetness and associated taste.

Under these circumstances, a stevia plant containing 5.33% or 5.71 % of RebE per dried leaf is known (Patent Literature 3).

### CITATION LIST

### PATENT LITERATURE

Patent Literature 1: WO2007/070224
Patent Literature 2: WO2010/038911
Patent Literature 3: WO2018/102648

### SUMMARY OF THE INVENTION

### TECHNICAL PROBLEM

Much remains unknown about gene information or what kind of gene is involved in the control of in vivo events in stevia, for example. Thus, it is desired to further elucidate gene information on stevia.

### MEANS FOR SOLVING THE PROBLEMS

The present invention provides a stevia plant having specific single nucleotide polymorphism (SNP) in the genomic nucleotide sequence, a method of producing the plant, and a method of screening for the plant. The feature of such a stevia plant is that the content of rebaudioside E is high.

In one aspect, the present invention provides the following.
[1] A stevia plant having at least one of the following genetic features (A) to (E).
   (A) Homozygous or heterozygous for the allele wherein the base at the position corresponding to position 40 of SEQ ID NO: 1 is C.
   (B) Homozygous or heterozygous for the allele wherein the base at the position corresponding to position 21 of SEQ ID NO: 2 is T.
   (C) Homozygous or heterozygous for the allele wherein the base at the position corresponding to position 28 of SEQ ID NO: 3 is T.
   (D) Homozygous or heterozygous for the allele wherein the base at the position corresponding to position 59 of SEQ ID NO: 4 is C.
   (E) Homozygous or heterozygous for the allele wherein the base at the position corresponding to position 64 of SEQ ID NO: 5 is T.
[2] The plant according to [1] above, comprising 0.07 % or more of rebaudioside E per unit mass of a dried leaf.
[3] The plant according to [1] or [2] above, wherein the plant is a non-genetically modified plant.
[4] The plant according to any one of [1] to [3] above, wherein the plant includes a stevia plant subjected to a mutagenesis treatment and a progeny plant thereof.
[5] A seed, a dried leaf, a tissue, a tissue culture or a cell of the plant according to any one of [1] to [4] above.
[6] The tissue, tissue culture or cell according to [5] above, which is selected from an embryo, a meristem cell, a pollen, a leaf, a root, a root apex, a petal, a protoplast, a leaf section and a callus.
[7] A method of producing a high rebaudioside E-content stevia plant comprising 0.07 % or more of rebaudioside E per unit mass of a dried leaf, the method comprising a step of crossing the stevia plant according to any one of [1] to [4] above with a second stevia plant.
[8] The method according to [7], wherein the second plant is the stevia plant according to any one of [1] to [4].
[9] An extract of the plant according to any one of [1] to [4], or of the seed, dried leaf, tissue, tissue culture or cell according to [5] above, wherein the extract comprises rebaudioside E.
[10] A food or beverage, a sweetener composition, a flavor or a medicament, comprising the extract according to [9] above.
[11] A method of producing an extract comprising rebaudioside E, comprising a step of obtaining an extract comprising rebaudioside E from the plant according to any one of [1] to [4], or from the seed, dried leaf, tissue, tissue culture or cell according to [5],
[12] A method of producing rebaudioside E, comprising a step of purifying rebaudioside E from the extract comprising rebaudioside E according to [11] above.
[13] A method of producing a food or beverage, a sweetener composition, a flavor or a medicament, comprising:
   a step of providing an extract of the plant according to any one of [1] to [4] above, an extract of the seed, dried leaf, tissue, tissue culture or cell according to [5], or the extract according to [11]; and
   a step of adding the extract to a raw material for the food or beverage, sweetener composition, flavor or medicament.
[14] A method of screening for the stevia plant according to any one of [1] to [4] above, comprising a step of detecting from the genome of a test plant the presence and/or the absence of at least one of the following genetic features (A) to (E).
   (A) Homozygous or heterozygous for the allele wherein the base at the position corresponding to position 40 of SEQ ID NO: 1 is C.
   (B) Homozygous or heterozygous for the allele wherein the base at the position corresponding to position 21 of SEQ ID NO: 2 is T.
   (C) Homozygous or heterozygous for the allele wherein the base at the position corresponding to position 28 of SEQ ID NO: 3 is T.
   (D) Homozygous or heterozygous for the allele wherein the base at the position corresponding to position 59 of SEQ ID NO: 4 is C.
   (E) Homozygous or heterozygous for the allele wherein the base at the position corresponding to position 64 of SEQ ID NO: 5 is T.
[15] The method according to [14] above, further comprising a step of measuring the content of rebaudioside E in a leaf tissue.
[16] The method according to [14] or [15], wherein the plant obtained by the screening comprises 0.07 % or more of rebaudioside E per unit mass of a dried leaf.

### ADVANTAGEOUS EFFECTS OF INVENTION

The present invention enables the obtainment of a stevia plant having a particular SNP in the nucleotide sequence of the genome and high rebaudioside E content, and the provision of an approach for producing such a plant, a leaf obtainable from such a plant, and a food, a drink, etc. containing the useful steviol glycoside obtained from this leaf.

### BRIEF DESCRIPTION OF DRAWINGS

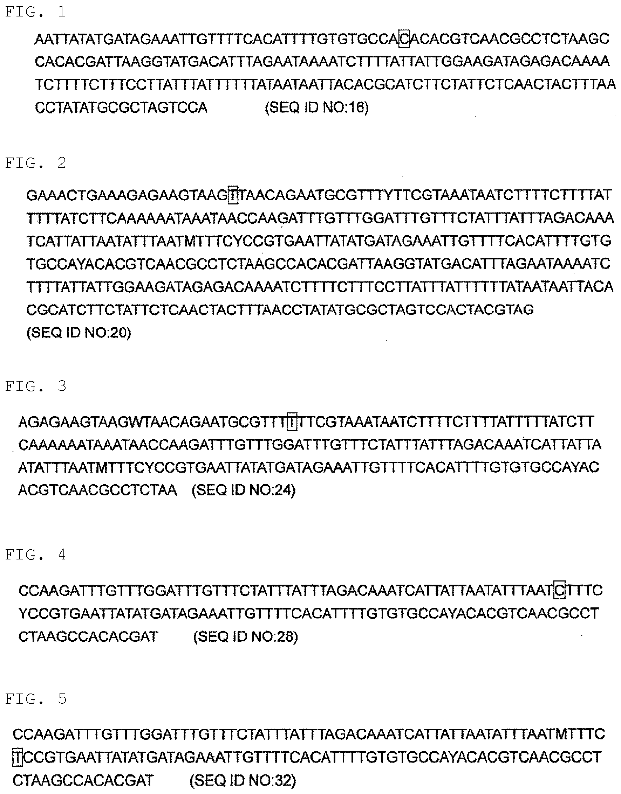

Figure 1 is a diagram showing the variation (A) in the nucleotide sequence of SEQ ID NO: 16. The boxed base is a base related to the variation (A).
Figure 2 is a diagram showing the variation (B) in the nucleotide sequence of SEQ ID NO: 20. The boxed base is a base related to the variation (B).
Figure 3 is a diagram showing the variation (C) in the nucleotide sequence of SEQ ID NO: 24. The boxed base is a base related to the variation (C).
Figure 4 is a diagram showing the variation (D) in the nucleotide sequence of SEQ ID NO: 28. The boxed base is a base related to the variation (D).
Figure 5 is a diagram showing the variation (E) in the nucleotide sequence of SEQ ID NO: 32. The boxed base is a base related to the variation (E).

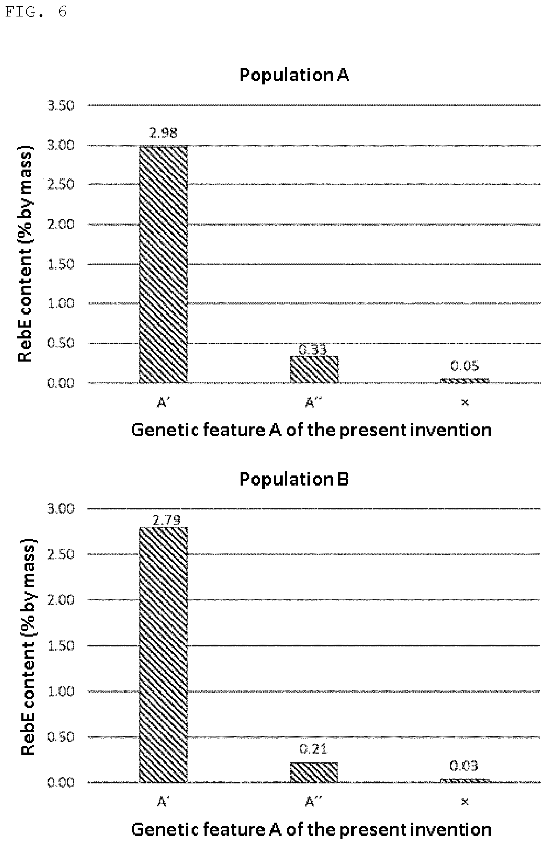

Figure 6 shows the relationship between the genetic feature (A) and an average RebE content in Examples.
Figure 7 is a diagram showing results of the analysis on whether a stevia plant has a genetic feature (A) by dCAPS method. Lane 1 shows electrophoretic images of samples of DNA of individuals homozygous for alleles with variation (A) treated with restriction-enzyme RsaI, lane 2 shows electrophoretic images of samples of DNA of individuals heterozygous for alleles with variation (A) treated with restriction-enzyme RsaI, and lane 3 shows DNA of individuals homozygous for alleles without variation (A) treated with restriction-enzyme RsaI.

### DESCRIPTION OF EMBODIMENTS

Hereinafter, the present invention will be described in detail. The embodiments are given below merely for illustrating the present invention and are not intended to limit the present invention by such embodiments. The present invention can be carried out in various modes without departing from the spirit of the present invention.

Note that all documents, as well as laid-open application publications, patent application publications, and other patent documents cited herein shall be incorporated herein by reference. The present specification incorporates the contents of the specification and the drawings of Japanese Patent Application No. 2020-084130, filed on May 12, 2020, from which the present application claims priority.

### 1. Stevia plant

In one embodiment, the present invention provides a stevia plant (hereinafter, may be generically referred to as the "plant of the present invention" or "stevia plant of the present invention") derived from a wild type stevia plant and having at least one of the following genetic features (A) to (E) (hereinafter, the at least one of the genetic features (A) to (E) may be generically referred to as the "genetic feature of the present invention").
(A) Homozygous or heterozygous for the allele wherein the base at the position corresponding to position 40 of SEQ ID NO: 1 is C (hereinafter, may be referred to as the "genetic feature (A) of the present invention").
(B) Homozygous or heterozygous for the allele wherein the base at the position corresponding to position 21 of SEQ ID NO: 2 is T (hereinafter, may be referred to as the "genetic feature (B) of the present invention").
(C) Homozygous or heterozygous for the allele wherein the base at the position corresponding to position 28 of SEQ ID NO: 3 is T (hereinafter, may be referred to as the "genetic feature (C) of the present invention").
(D) Homozygous or heterozygous for the allele wherein the base at the position corresponding to position 59 of SEQ ID NO: 4 is C (hereinafter, may be referred to as the "genetic feature (D) of the present invention").
(E) Homozygous or heterozygous for the allele wherein the base at the position corresponding to position 64 of SEQ ID NO: 5 is T (hereinafter, may be referred to as the "genetic feature (E) of the present invention").

In a preferable embodiment, the stevia plant of the present invention has the genetic feature (A). In a more preferable embodiment, the stevia plant of the present invention has a genetic feature of being homozygous for the allele wherein the base at the position corresponding to position 40 of SEQ ID NO: 1 is C (hereinafter, may be referred to as the "genetic feature (A') of the present invention") among the genetic feature (A) of the present invention. A genetic feature of being heterozygous for the allele wherein the base at the position corresponding to position 40 of SEQ ID NO: 1 is C may be referred to as the genetic feature (A") of the present invention.

As the genetic feature (B) of the present invention, a genetic feature of being homozygous for the allele wherein the base at the position corresponding to position 21 of SEQ ID NO: 2 is T (hereinafter, may be referred to as the "genetic feature (B') of the present invention") is preferred. A genetic feature of being heterozygous for the allele wherein the base at the position corresponding to position 21 of SEQ ID NO: 2 is T may be referred to as the genetic feature (B") of the present invention.

As the genetic feature (C) of the present invention, a genetic feature of being homozygous for the allele wherein the base at the position corresponding to position 28 of SEQ ID NO: 3 is T (hereinafter, may be referred to as the "genetic feature (C') of the present invention") is preferred. A genetic feature of being heterozygous for the allele wherein the base at the position corresponding to position 28 of SEQ ID NO: 3 is T may be referred to as the genetic feature (C") of the present invention.

As the genetic feature (D) of the present invention, a genetic feature of being homozygous for the allele wherein the base at the position corresponding to position 59 of SEQ ID NO: 4 is C (hereinafter, may be referred to as the "genetic feature (D') of the present invention") is preferred. A genetic feature of being heterozygous for the allele wherein the base at the position corresponding to position 59 of SEQ ID NO: 4 is C may be referred to as the genetic feature (D") of the present invention.

As the genetic feature (E) of the present invention, a genetic feature of being homozygous for the allele wherein the base at the position corresponding to position 64 of SEQ ID NO: 5 is T (hereinafter, may be referred to as the "genetic feature (E') of the present invention") is preferred. A genetic feature of being heterozygous for the allele wherein the base at the position corresponding to position 64 of SEQ ID NO: 5 is T may be referred to as the genetic feature (E") of the present invention.

In some embodiments, the plant of the present invention may have a plurality of the above genetic features. In these embodiments, the number of genetic features may be any of 2 to 5, i.e., 2, 3, 4 or 5. The combination of the genetic features is not particularly limited and includes, for example, each of the following combinations within the parentheses: (A, B), (A, C), (A, D), (A, E), (B, C), (B, D), (B, E), (C, D), (C, E), (D, E), (A, B, C), (A, B, D), (A, B, E), (A, C, D), (A, C, E), (A, D, E), (B, C, D), (B, C, E), (B, D, E), (C, D, E), (A, B, C, D), (A, B, C, E), (A, B, D, E), (A, C, D, E), (B, C, D, E), (A, B, C, D, E), (A', B), (A', C), (A', D), (A', E), (A', B, C), (A', B, D), (A', B, E), (A', C, D), (A', C, E), (A', D, E), (A', B, C, D), (A', B, C, E), (A', B, D, E), (A', C, D, E), (A', B, C, D, E), (A', B'), (A', C'), (A', D'), (A', E'), (B', C'), (B', D'), (B', E'), (C', D'), (C', E'), (D', E'), (A', B', C'), (A', B', D'), (A', B', E'), (A', C', D'), (A', C', E'), (A', D', E'), (B', C', D'), (B', C', E'), (B', D', E'), (C', D', E'), (A', B', C', D'), (A', B', C', E'), (A', B', D', E'), (A', C', D', E'), (B', C', D', E'), (A', B', C', D', E'). In the above, for example, (A, B) means the combination of the genetic feature (A) and the genetic feature (B).

In a preferable embodiment, the plant of the present invention has the genetic feature (A). In a more preferable embodiment, the plant of the present invention has the genetic feature (A'). In another preferable embodiment, the plant of the present invention has the genetic feature (A) and has at least one of the genetic features (B) to (E). In a more preferable embodiment, the plant of the present invention has the genetic feature (A) and has all of the genetic features (B) to (E). In an alternative preferable embodiment, the plant of the present invention has the genetic feature (A) and has at least one of the genetic features (B) to (D). In a more preferable embodiment, the plant of the present invention has the genetic feature (A) and has all of the genetic features (B) to (D). In an alternative preferable embodiment, the plant of the present invention has the genetic feature (A') and has at least one of the genetic features (B) to (E). In a more preferable embodiment, the plant of the present invention has the genetic feature (A') and has all of the genetic features (B) to (E). In an alternative preferable embodiment, the plant of the present invention has the genetic feature (A') and has at least one of the genetic features (B) to (D). In a more preferable embodiment, the plant of the present invention has the genetic feature (A') and has all of the genetic features (B) to (D). In an alternative preferable embodiment, the plant of the present invention has the genetic feature (A') and has at least one of the genetic features (B') to (E'). In a more preferable embodiment, the plant of the present invention has the genetic feature (A') and has all of the genetic features (B') to (E'). In an alternative preferable embodiment, the plant of the present invention has the genetic feature (A') and has at least one of the genetic features (B') to (D'). In a more preferable embodiment, the plant of the present invention has the genetic feature (A') and has all of the genetic features (B') to (D').

The phrase "position corresponding to" means the following. In case a sequence identical to a reference sequence (e.g., SEQ ID NOs: 1 to 5, etc.) is present in the genome, it means a position in the sequence (e.g., 40, 21, 28, 59, 64, etc.) present in the genome, and in case a sequence identical to the reference sequence is not present in the genome, it means a position in a sequence in the genome corresponding to the reference sequence, which corresponds to the position in the reference sequence. Whether or not a sequence identical to or corresponding to the reference sequence exists in the genome can be determined by, for example, amplifying genomic DNA of the stevia plant of interest with a primer capable of amplifying the reference sequence by PCR, sequencing the amplified product, and performing alignment analysis between the obtained sequence and the reference sequence. Non-limiting examples of a sequence corresponding to a reference sequence include, for example, a nucleotide sequence having a sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1% or more, 98.4% or more, 98.7% or more, 99% or more, 99.2% or more, 99.5% or more, or 99.8% or more to the reference sequence. The position corresponding to the position in the reference sequence in the sequence corresponding to the reference sequence in the genome can be determined by taking into account the nucleotide sequence before and after the position in the reference sequence and the like. For example, a position in the sequence corresponding to the reference sequence in the genome corresponding to a position in the reference sequence can be determined by an alignment analysis of a reference sequence with a sequence corresponding to a reference sequence in the genome.

For instance, when taking "the position corresponding to position 40 of SEQ ID NO: 1" of the genetic feature (A) of the present invention as an example, in case the genome of a stevia plant has a portion consisting of a nucleotide sequence identical to SEQ ID NO: 1, "the position corresponding to position 40 of SEQ ID NO: 1" is position 40 from the 5' end of the portion consisting of a nucleotide sequence identical to SEQ ID NO: 1 in the genome. On the other hand, in case the genome of a stevia plant has a portion consisting of a nucleotide sequence which is not identical to, but which corresponds to SEQ ID NO: 1, the genome does not have a portion consisting of a nucleotide sequence identical to SEQ ID NO: 1. Therefore, "the position corresponding to position 40 of SEQ ID NO: 1" does not necessarily correspond to position 40 from the 5' end of the portion corresponding to SEQ ID NO: 1. However, it is possible to identify "the position corresponding to position 40 of SEQ ID NO: 1" in the genome of such a stevia plant by taking into account the nucleotide sequence before and after the position 40 of SEQ ID NO: 1, and the like. For instance, one can identify "the position corresponding to position 40 of SEQ ID NO: 1" in the genome of a stevia plant by an alignment analysis of the nucleotide sequence of a portion corresponding to SEQ ID NO: 1 in the genome of a stevia plant and the nucleotide sequence of SEQ ID NO: 1.

Here, a position selected from the group consisting of (A) a position corresponding to position 40 of SEQ ID NO: 1, (B) a position corresponding to position 21 of SEQ ID NO: 2, (C) a position corresponding to position 28 of SEQ ID NO: 3, (D) a position corresponding to position 59 of SEQ ID NO: 4, and (E) a position corresponding to position 64 of SEQ ID NO: 5 may be generically referred to as a "polymorphic site of the present invention" or a "variation site of the present invention". Also, each of the above positions (A) to (E) may be referred to as a "polymorphic site (A) of the present invention", a "polymorphic site (B) of the present invention", a "variation site (A) of the present invention", a "variation site (B) of the present invention", or the like.

"The portion consisting of a nucleotide sequence corresponding to SEQ ID NO: 1" means, for instance, a portion consisting of a nucleotide sequence having a sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1% or more, 98.4% or more, 98.7% or more, 99% or more, 99.2% or more, 99.5% or more, or 99.8% or more to the nucleotide sequence of SEQ ID NO: 1.

In one embodiment, "the portion consisting of a nucleotide sequence corresponding to SEQ ID NO: 1" includes a portion of the genome of a stevia plant which can be amplified by PCR using a forward primer which hybridizes to a complementary sequence of a portion of positions 1 to 39 from the 5' end of SEQ ID NO: 1 (i.e., from the 5' end of SEQ ID NO: 1 to the base upstream by one base relative to the variation site (A)) and a reverse primer which hybridizes to a portion of positions 1 to 163 from the 3' end of SEQ ID NO: 1 (i.e., from the 3' end of SEQ ID NO: 1 to the base downstream by one base relative to the variation site (A)).

For simplicity, the genetic feature (A) of the present invention is used here as an example for explanation, but the same applies to the genetic features (B) to (E) of the present invention.

In a specific embodiment, "the portion consisting of a nucleotide sequence corresponding to SEQ ID NO: 1" includes, for instance, a portion of the genome of a stevia plant which can be amplified by PCR using a forward primer comprising the nucleotide sequence of SEQ ID NO: 6 and a reverse primer comprising the nucleotide sequence of SEQ ID NO: 7.

In a specific embodiment, "the portion consisting of a nucleotide sequence corresponding to SEQ ID NO: 2" includes, for instance, a portion of the genome of a stevia plant which can be amplified by PCR using a forward primer comprising the nucleotide sequence of SEQ ID NO: 8 and a reverse primer comprising the nucleotide sequence of SEQ ID NO: 9.

In a specific embodiment, "the portion consisting of a nucleotide sequence corresponding to SEQ ID NO: 3" includes, for instance, a portion of the genome of a stevia plant which can be amplified by PCR using a forward primer comprising the nucleotide sequence of SEQ ID NO: 10 and a reverse primer comprising the nucleotide sequence of SEQ ID NO: 11.

In a specific embodiment, "the portion consisting of a nucleotide sequence corresponding to SEQ ID NO: 4" includes, for instance, a portion of the genome of a stevia plant which can be amplified by PCR using a forward primer comprising the nucleotide sequence of SEQ ID NO: 12 and a reverse primer comprising the nucleotide sequence of SEQ ID NO: 13.

In a specific embodiment, "the portion consisting of a nucleotide sequence corresponding to SEQ ID NO: 5" includes, for instance, a portion of the genome of a stevia plant which can be amplified by PCR using a forward primer comprising the nucleotide sequence of SEQ ID NO: 14 and a reverse primer comprising the nucleotide sequence of SEQ ID NO: 15.

In a specific embodiment, "the allele wherein the base at the position corresponding to position 40 of SEQ ID NO: 1 is C" comprises the nucleotide sequence of SEQ ID NO: 16, 17, 18 or 19.

In a specific embodiment, "the allele wherein the base at the position corresponding to position 21 of SEQ ID NO: 2 is T" comprises the nucleotide sequence of SEQ ID NO: 20, 21, 22 or 23.

In a specific embodiment, "the allele wherein the base at the position corresponding to position 28 of SEQ ID NO: 3 is T" comprises the nucleotide sequence of SEQ ID NO: 24, 25, 26 or 27.

In a specific embodiment, "the allele wherein the base at the position corresponding to position 59 of SEQ ID NO: 4 is C" comprises the nucleotide sequence of SEQ ID NO: 28, 29, 30 or 31.

In a specific embodiment, "the allele wherein the base at the position corresponding to position 64 of SEQ ID NO: 5 is T" comprises the nucleotide sequence of SEQ ID NO: 32, 33, 34 or 35.

Also, a variation selected from the group consisting of (A) a variation from T to C at a position corresponding to position 40 of SEQ ID NO: 1, (B) a variation from A to T at a position corresponding to position 21 of SEQ ID NO: 2, (C) a variation from C to T at a position corresponding to position 28 of SEQ ID NO: 3, (D) a variation from A to C at a position corresponding to position 59 of SEQ ID NO: 4, and (E) a variation from C to T at a position corresponding to position 64 of SEQ ID NO: 5 may be generically referred to as a "polymorphism of the present invention" or a "variation of the present invention". Also, each of the above variations (A) to (E) may be referred to as a "polymorphism (A) of the present invention", "polymorphism (B) of the present invention", a "variation (A) of the present invention", a "variation (B) of the present invention", or the like.

The above genetic features can be detected by PCR method, TaqMan PCR method, sequencing method, microarray method, Invader method, TILLING method, RAD (random amplified polymorphic DNA) method, restriction fragment length polymorphism (RFLP) method, PCR-SSCP method, AFLP (amplified fragment length polymorphism) method, SSLP (simple sequence length polymorphism) method, CAPS (cleaved amplified polymorphic sequence) method, dCAPS (derived cleaved amplified polymorphic sequence) method, allele-specific oligonucleotide (ASO) method, ARMS method, denaturing gradient gel electrophoresis (DGGE) method, CCM (chemical cleavage of mismatch) method, DOL method, MALDI-TOF/MS method, TDI method, padlock probe method, molecular beacon method, DASH (dynamic allele specific hybridization) method, UCAN method, ECA method, PINPOINT method, PROBE (primer oligo base extension) method, VSET (very short extension) method, Survivor assay, Sniper assay, Luminex assay, GOOD method, LCx method, SNaPshot method, Mass ARRAY method, pyrosequencing method, SNP-IT method, melting curve analysis method, etc., but detection methods are not limited thereto.

In a specific embodiment, each genetic feature of the present invention is detectable by using the following combination of a primer set and a restriction enzyme.

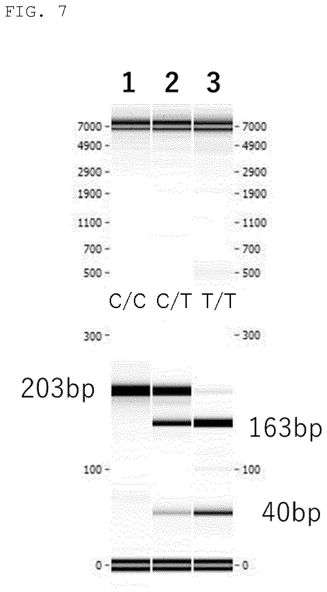

In case a candidate plant has the variation (A), for example, only a band of approximately 203 bp long (e.g., SEQ ID NO: 38) is obtained by: performing PCR amplification using a forward primer having the nucleotide sequence shown in SEQ ID NO: 36 and a reverse primer having the nucleotide sequence shown in SEQ ID NO: 37 on the genomic DNA of the candidate plant; and treating the obtained PCR product (approximately 203 bp long, e.g., SEQ ID NO: 38) with a restriction enzyme RsaI. On the other hand, in case the candidate plant does not have the variation (A) (the base at the position corresponding to position 40 of SEQ ID NO: 1 is T), a band of approximately 40 bp long (e.g., SEQ ID NO: 40) and a band of approximately 163 bp long (e.g., SEQ ID NO: 41) are obtained by: performing PCR amplification in the same way as above; and treating the obtained PCR product (approximately 203 bp long, e.g., SEQ ID NO: 39) with a restriction enzyme RsaI. An example in which the variation (A) was detected by the above approach is shown in Figure 7. Lane 1 was loaded with a sample prepared by treating DNA of a stevia individual homozygous for the allele having the variation (A) with the restriction enzyme RsaI. Lane 2 was loaded with a sample prepared by treating DNA of a stevia individual heterozygous for the allele having the variation (A) with the restriction enzyme RsaI. Lane 3 was loaded with a sample prepared by treating DNA of a stevia individual homozygous for the allele not having the variation (A) with the restriction enzyme RsaI. The band of approximately 203 bp long without enzymatic degradation are found in the lane 1. The band of approximately 40 bp long and the band of approximately 163 bp long from enzymatic degradation, and the band of approximately 203 bp long without enzymatic degradation are found in the lane 2. Thus, the candidate plant can be determined as having the genetic feature (A) of the present invention, when the band of approximately 203 bp long derived from a non-degraded product is found by the above dCAPS method. The candidate plant can be determined as having the genetic feature (A') of the present invention, when the band of approximately 40 bp long and/or the band of approximately 163 bp long derived from a degraded product are/is not found and the band of approximately 203 bp long derived from a non-degraded product is found. The candidate plant can be determined as having the genetic feature (A") of the present invention, when the band of approximately 40 bp long and/or the band of approximately 163 bp long derived from a degraded product, and the band of approximately 203 bp long derived from a non-degraded product are found.

In case a candidate plant has the variation (B), for example, a band of approximately 18 bp long (e.g., SEQ ID NO: 46) and a band of approximately 346 bp long (e.g., SEQ ID NO: 47) are obtained by: performing PCR amplification using a forward primer having the nucleotide sequence shown in SEQ ID NO: 42 and a reverse primer having the nucleotide sequence shown in SEQ ID NO: 43 on the genomic DNA of the candidate plant; and treating the obtained PCR product (approximately 364 bp long, e.g., SEQ ID NO: 44) with a restriction enzyme AclI. On the other hand, in case the candidate plant does not have the variation (B), only a band of approximately 364 bp long (e.g., SEQ ID NO: 45) is obtained by: performing PCR amplification in the same way as above; and treating the obtained PCR product (approximately 364 bp long, e.g., SEQ ID NO: 45) with a restriction enzyme AclI. Thus, the candidate plant can be determined as having the genetic feature (B) of the present invention, when the band of approximately 18 bp long and/or the band of approximately 346 bp long derived from a degraded product are/is found by the above dCAPS method. The candidate plant can be determined as having the genetic feature (B') of the present invention, when the band of approximately 18 bp long and/or the band of approximately 346 bp long derived from a degraded product are/is found and the band of approximately 364 bp long derived from a non-degraded product is not found. The candidate plant can be determined as having the genetic feature (B") of the present invention, when the band of approximately 18 bp long and/or the band of approximately 346 bp long derived from a degraded product, and the band of approximately 364 bp long derived from a non-degraded product are found.

In case a candidate plant has the variation (C), for example, a band of approximately 19 bp long (e.g., SEQ ID NO: 52) and a band of approximately 334 bp long (e.g., SEQ ID NO: 53) are obtained by: performing PCR amplification using a forward primer having the nucleotide sequence shown in SEQ ID NO: 48 and a reverse primer having the nucleotide sequence shown in SEQ ID NO: 49 on the genomic DNA of the candidate plant; and treating the obtained PCR product (approximately 353 bp long, e.g., SEQ ID NO: 50) with a restriction enzyme AluI. On the other hand, in case the candidate plant does not have the variation (C), only a band of approximately 353 bp long (e.g., SEQ ID NO: 51) is obtained by: performing PCR amplification in the same way as above; and treating the obtained PCR product (approximately 353 bp long, e.g., SEQ ID NO: 51) with a restriction enzyme AluI. Thus, the candidate plant can be determined as having the genetic feature (C) of the present invention, when the band of approximately 19 bp long and/or the band of approximately 334 bp long derived from a degraded product are/is found by the above dCAPS method. The candidate plant can be determined as having the genetic feature (C') of the present invention, when the band of approximately 19 bp long and/or the band of approximately 334 bp long derived from a degraded product are/is found and the band of approximately 353 bp long derived from a non-degraded product is not found. The candidate plant can be determined as having the genetic feature (C") of the present invention, when the band of approximately 19 bp long and/or the band of approximately 334 bp long derived from a degraded product, and the band of approximately 353 bp long derived from a non-degraded product are found.

In case a candidate plant has the variation (D), for example, a band of approximately 21 bp long (e.g., SEQ ID NO: 58) and a band of approximately 328 bp long (e.g., SEQ ID NO: 59) are obtained by: performing PCR amplification using a forward primer having the nucleotide sequence shown in SEQ ID NO: 54 and a reverse primer having the nucleotide sequence shown in SEQ ID NO: 55 on the genomic DNA of the candidate plant; and treating the obtained PCR product (approximately 349 bp long, e.g., SEQ ID NO: 56) with a restriction enzyme AluI. On the other hand, in case the candidate plant does not have the variation (D), only a band of approximately 349 bp long (e.g., SEQ ID NO: 57) is obtained by: performing PCR amplification in the same way as above; and treating the obtained PCR product (approximately 349 bp long, e.g., SEQ ID NO: 57) with a restriction enzyme AluI. Thus, the candidate plant can be determined as having the genetic feature (D) of the present invention, when the band of approximately 21 bp long and/or the band of approximately 328 bp long derived from a degraded product are/is found by the above dCAPS method. The candidate plant can be determined as having the genetic feature (D') of the present invention, when the band of approximately 21 bp long and/or the band of approximately 328 bp long derived from a degraded product are/is found and the band of approximately 349 bp long derived from a non-degraded product is not found. The candidate plant can be determined as having the genetic feature (D") of the present invention, when the band of approximately 21 bp long and/or the band of approximately 328 bp long derived from a degraded product, and the band of approximately 349 bp long derived from a non-degraded product are found.

In case a candidate plant has the variation (E), for example, a band of approximately 21 bp long (e.g., SEQ ID NO: 64) and a band of approximately 328 bp long (e.g., SEQ ID NO: 65) are obtained by: performing PCR amplification using a forward primer having the nucleotide sequence shown in SEQ ID NO: 60 and a reverse primer having the nucleotide sequence shown in SEQ ID NO: 61 on the genomic DNA of the candidate plant; and treating the obtained PCR product (approximately 349 bp long, e.g., SEQ ID NO: 62) with a restriction enzyme AluI. On the other hand, in case the candidate plant does not have the variation (E), only a band of approximately 349 bp long (e.g., SEQ ID NO: 63) is obtained by: performing PCR amplification in the same way as above; and treating the obtained PCR product (approximately 349 bp long, e.g., SEQ ID NO: 63) with a restriction enzyme AluI. Thus, the candidate plant can be determined as having the genetic feature (E) of the present invention, when the band of approximately 21 bp long and/or the band of approximately 328 bp long derived from a degraded product are/is found by the above dCAPS method. The candidate plant can be determined as having the genetic feature (E') of the present invention, when the band of approximately 21 bp long and/or the band of approximately 328 bp long derived from a degraded product are/is found and the band of approximately 349 bp long derived from a non-degraded product is not found. The candidate plant can be determined as having the genetic feature (E") of the present invention, when the band of approximately 21 bp long and/or the band of approximately 328 bp long derived from a degraded product, and the band of approximately 349 bp long derived from a non-degraded product are found.

The term "approximately" as to bp long described above means ± 5 bp. The restriction enzyme treatment can be performed according to conditions recommended by the distributor of each restriction enzyme used.

The variation sites (A) to (E) of the present invention are positioned as adjacent as within 173 bp to each other on the genome and are in linkage disequilibrium. Thus, the plant of the present invention having any of the genetic features (A) to (E) tends to have the other genetic features (A) to (E). The plant of the present invention having any of the genetic features (A') to (E') tends to have the other genetic features (A') to (E').

The stevia plant having the genetic feature(s) of the present invention has a chemical feature of comprising 0.07% or more of RebE per unit mass of a dried leaf.

It is known that RebE has high relative sweetness, which is 1.5 or more times the sweetness of stevioside, for example, and has good quality of taste. Furthermore, RebE is reportedly highly soluble and stable. Sweeteners containing RebE are awaited as sweeteners having good palatability and quality of taste. Hence, high RebE content stevia plants are awaited.

The presence of the genetic features (A) to (E) of the present invention is highly related to the presence of the chemical feature of the present invention. Hence, a stevia plant having at least one of the genetic features (A) to (E) of the present invention is likely to have the chemical feature of the present invention, and a stevia plant having the chemical feature of the present invention may be screened for by using at least one of the genetic features (A) to (E) of the present invention as an index.

In the stevia plant of the present invention, the feature "the content of RebE is 0.07% or more per unit mass of a dried leaf' means that, for example, RebE is contained at a ratio of 0.07 % by mass or more (e.g., 0.035 mg or more) in a dried leaf having a predetermined mass (e.g., 50 mg). In this embodiment, the ratio of RebE per unit mass of a dried leaf is not limited and may be, for example,0.07 % or more, more than 0.10 %, 0.15 % or more, more than 0.49 %, 0.57 % or more, more than 0.62 %, 1.07 % or more, 1.57 % or more, 1.74 % or more, 2.07 % or more, 2.10 % or more, 2.57 % or more, 3.07 % or more, 3.57 % or more, 4.07 % or more, 4.57 % or more, 5.07 % or more, 5.57 % or more, 6.07 % or more. The upper limit of the ratio of RebE per unit mass of a dried leaf is not particularly limited and may be, for example, 20 %, 15 %, 10% or 7%.

In this context, the dried leaf refers to a leaf having a water content decreased to 3 to 4% by weight by drying a fresh leaf of the stevia plant of the present invention.

In one embodiment, the plant of the present invention has a mass ratio of RebE to the total steviol glycoside (TSG) of 0.5 % or more. This means that, for example, when the mass of RebE contained in a leaf (e.g., a dried leaf or a fresh leaf) is indicated by RebE/TSG % as the ratio to the total mass of steviol glycosides obtained from the leaf, the value of RebE/TSG is 0.5 % or more. In this embodiment, the value of RebE/TSG is not limited and may be, for example, 0.5 % or more, 1.5 % or more, 2.0 % or more, 2.4 % or more, 2.5 % or more, 3.0 % or more, 3.5 % or more, 4.0 % or more, 4.5 % or more, 5.0 % or more, 5.5 % or more, 6.0 % or more, 6.5 % or more, 7.0 % or more, 7.5 % or more, 8.0 % or more, 8.5 % or more, 9.0 % or more, 9.5 % or more, 10.0 % or more, 15.0 % or more, 20.0 % or more, 22.4 % or more, 25.0 % or more, 25.5 % or more, 30.0 % or more, 33.5 % or more, 35.0 % or more, 40.0 % or more, 45.0 % or more. The upper limit of the mass ratio of RebE to TSG is not particularly limited and may be, for example, 85 %, 75 %, 65 % or 55 %.

TSG is a generic name for measurable steviol glycosides and includes neither an unknown steviol glycoside nor a steviol glycoside present at a level less than the detection limit. Preferably, the TSG is any combination of two or more members selected from the group consisting of RebA, RebB, RebC, RebD, RebE, RebF, RebG, RebI, RebJ, RebK, RebM, RebN, RebO, RebQ, RebR, dulcoside A, rubusoside, steviolmonoside, steviolbioside and stevioside. For examples, in certain embodiment, the TSG consists of RebA, RebB, RebC, RebD, RebE, RebF, RebG, RebM, RebN and stevioside.

In one embodiment, the plant of the present invention has a value of RebE/stevioside of 1 or less. This means that, for example, the ratio of the mass of RebE obtained from a leaf (e.g., a dried leaf or a fresh leaf) to the mass of stevioside obtained from the leaf (RebE/stevioside) is 1 or less. The value of RebE/stevioside is not limited and may be, for example, 1 or less, 0.95 or less, 0.90 or less, 0.85 or less, 0.65 or less, or the like. The lower limit of RebE/stevioside is not particularly limited and may be, for example, 0.07 or more, 0.11 or more, 0.12 or more, 0.13 or more, 0.14 or more, 0.15 or more, 0.17 or more, 0.18 or more, 0.20 or more, 0.22 or more, 0.25 or more, 0.27 or more, 0.28 or more, 0.30 or more, 0.32 or more, 0.35 or more, 0.38 or more, 0.37 or more, 0.50 or more, or the like.

The steviol glycosides such as RebE and stevioside can be extracted in the state of a liquid extract by reacting a fresh leaf or a dried leaf of the plant of the present invention with a suitable solvent (an aqueous solvent such as water or an organic solvent such as an alcohol, ether or acetone). For the extraction conditions, etc., see a method described in Ohta et al., J. Appl. Glycosci., Vol. 57, No. 3, 199-209 (2010) or WO2010/038911, or a method described in Examples mentioned later.

Individual steviol glycosides, for example, RebE, can be further purified from the liquid extract thus obtained by use of a method known in the art such as a gradient of ethyl acetate or any of other organic solvents:water, high performance liquid chromatography (HPLC), gas chromatography, time-of-flight mass spectrometry (TOF-MS), or ultra (high) performance liquid chromatography (UPLC).

The contents of the steviol glycosides such as RebE and stevioside can be measured by a method described in Ohta et al., supra or WO2010/038911, or a method described in Examples mentioned later. Specifically, for instance, a fresh leaf can be sampled from the stevia plant of the present invention, followed by measurement by LC-MS/MS and the like.

The plant of the present invention may be the one obtained by a genetic modification approach or a progeny thereof (hereinafter, may be referred to as "genetically modified plant") or the one obtained by a non-genetic modification approach or a progeny thereof (hereinafter, may be referred to as "non-genetically modified plant"). Examples of the "non-genetic modification approach" include, besides hybridization, selfing, and the like, a method of inducing a variation in the gene of a host cell (or a host plant) without transfection with a foreign gene. Examples of such a method include a method of allowing a mutagen to act on a plant cell. Examples of such a mutagen include ethyl methanesulfonate (EMS) and sodium azide. For example, EMS can be used at a concentration such as 0.1%, 0.2%, 0.3%, 0.4%, 0.5%, 0.6%, 0.7%, 0.8%, 0.9%, or 1.0% to treat a plant cell. The treatment time is 1 to 48 hours, 2 to 36 hours, 3 to 30 hours, 4 to 28 hours, 5 to 26 hours, or 6 to 24 hours. The procedures themselves of the treatment are known in the art and can be performed by dipping a water-absorbed seed obtained through a water absorption process in a treatment solution containing the mutagen at the concentration described above for the treatment time described above.

An alternative example of the non-genetic modification approach can be a method of irradiating a plant cell with radiation or light beam such as X-ray, y ray, or ultraviolet ray. In this case, a cell irradiated using an appropriate dose (ultraviolet lamp intensity, distance, and time) of ultraviolet ray is cultured in a selective medium or the like, and then, a cell, a callus, or a plant having the trait of interest can be selected. In this operation, the irradiation intensity is 0.01 to 100 Gr, 0.03 to 75 Gr, 0.05 to 50 Gr, 0.07 to 25 Gr, 0.09 to 20 Gr, 0.1 to 15 Gr, 0.1 to 10 Gr, 0.5 to 10 Gr, or 1 to 10 Gr. The irradiation distance is 1 cm to 200 m, 5 cm to 100 m, 7 cm to 75 m, 9 cm to 50 m, 10 cm to 30 m, 10 cm to 20 m, or 10 cm to 10 m. The irradiation time is 1 minute to 2 years, 2 minutes to 1 year, 3 minutes to 0.5 years, 4 minutes to 1 month, 5 minutes to 2 weeks, or 10 minutes to 1 week. The irradiation intensity, distance and time differ depending on the type of radiation or the state of the subject to be irradiated (cell, callus, or plant) and can be appropriately adjusted by those skilled in the art.

Approaches such as cell fusion, anther culture (haploid induction), and remote crossing (haploid induction) are also known in the art.

In general, plant cells may involve a mutation during culture. Therefore, it is preferred to regenerate a plant individual, for more stably maintaining the trait.

The scope of the present invention does not exclude a plant obtained by the ex-post facto genetic recombination (e.g., genome editing) with the plant of the present invention as a host (e.g., a plant further provided with another trait by genetic recombination with the plant of the present invention as a host).

The plant of the present invention may include not only the whole plant but a plant organ (e.g., a leaf, a petal, a stem, a root, and a seed), a plant tissue (e.g., epidermis, phloem, soft tissue, xylem, vascular bundle, palisade tissue, and spongy tissue), various forms of plant cells (e.g., suspended cultured cells), a protoplast, a leaf section, a callus, and the like. The leaf may be a dried leaf.

The plant of the present invention may also include a cultured plant cell or a tissue culture. This is because the plant can be regenerated by culturing such a cultured plant cell or a tissue culture. Examples of a regenerable form of the plant of the present invention include, but are not limited to, embryos, meristem cells, pollens, leaves, roots, root apices, petals, protoplasts, leaf sections and calluses.

### 2. Method of producing plant of present invention

In an alternative aspect, the present invention provides a method of producing a stevia plant characterized in that it comprises 0.07 % or more of rebaudioside E per unit mass of a dried leaf, the method comprising a step of crossing the stevia plant of the present invention with a second stevia plant (hereinafter, may be referred to as the "production method of the present invention"). The stevia plant produced by the method has the same phenotype and genetic features as those of the plant of the present invention.

The amount of RebE, the amount of RebE with respect to the amount of stevioside, the amount of RebE with respect to the amount of TSG, the combination of each feature (chemical feature and/or genetic feature), and the like are as described in the section relating to the plant of the present invention.

In one embodiment, the plant obtained by the production method of the present invention has the combination of the features (chemical feature(s) and/or genetic feature(s)), etc. described in the section relating to the plant of the present invention.

In the production method of the present invention, "hybridizing" means that the plant of the present invention (first generation (S1)) is crossed with a second plant (S1) to obtain a progeny plant thereof (plant produced by the production method of the present invention (second generation (S2)). The hybridizing method is preferably backcross. The "backcross" is an approach of further crossing a progeny plant (S2) generated between the plant of the present invention and the second plant, with the plant of the present invention (i.e., a plant having the genetic feature(s) of the present invention) (S1) to produce a plant having the genetic feature(s) of the present invention. When the second plant (S1) for use in the production method of the present invention has the same phenotype and genetic features as those of the plant of the present invention, the crossing is substantially backcross.

Hybridization is preferably performed over two generations or more, but in case where the genetic feature is heterozygous, etc., a plant having a desired combination of genetic features may be obtained in one generation.

Alternatively, the plant of the present invention can also be produced by selfing. The selfing can be performed by the self-pollination of the stamen pollen of the plant of the present invention with the pistil of the plant of the present invention.

Since the plant produced by the production method of the present invention has the same phenotype and genetic features as those of the plant of the present invention, the plant produced by the production method of the present invention can be further crossed with a third stevia plant to produce a stevia plant having a phenotype equivalent to that of the plant of the present invention.

In an alternative embodiment, the plant of the present invention may be produced by regenerating a plant by the culture of the tissue culture or the cultured plant cell mentioned above. The culture conditions are the same as those for culturing a tissue culture or a cell of the wild type stevia plant and are known in the art (Protocols for in vitro cultures and secondary metabolite analysis of aromatic and medicinal plants, Method in molecular biology, vol. 1391, pp. 113-123).

In a further alternative embodiment, the plant of the present invention may be produced by introducing the variation of the present invention to the genome of a stevia plant. The introduction of the variation may be performed by a genetic modification approach or may be performed by a non-genetic modification approach. The "non-genetic modification approach" is as described in the section relating to the plant of the present invention.

### 3. Method of screening for plant of present invention

The plant of the present invention or the plant having the same phenotype and/or genetic feature as those of the plant of the present invention can be screened for by detecting the genetic feature(s) of the present invention from a tissue of this plant. In this context, "screening" means that the plant of the present invention is discriminated from the other plants to select the plant of the present invention.

Thus, in an alternative aspect, the present invention provides a method of screening for a stevia plant, comprising a step of detecting the presence and/or the absence of at least one of the genetic features (A) to (E) of the present invention from the genome of a test plant (hereinafter, may be referred to as the "screening method of the present invention").

In one embodiment, the genetic feature(s) to be detected is at least one of the genetic features (A) to (E). In a preferable embodiment, the genetic feature to be detected is the genetic feature (A), more preferably the genetic feature (A'). In another preferable embodiment, the genetic features to be detected are the genetic feature (A) and at least one of the genetic features (B) to (E), more preferably the genetic feature (A) and all of the genetic features (B) to (E). In an alternative preferable embodiment, the genetic features to be detected are the genetic feature (A) and at least one of the genetic features (B) to (D), more preferably the genetic feature (A) and all of the genetic features (B) to (D). In an alternative preferable embodiment, the genetic features to be detected are the genetic feature (A') and at least one of the genetic features (B) to (E), more preferably the genetic feature (A') and all of the genetic features (B) to (E). In an alternative preferable embodiment, the genetic features to be detected are the genetic feature (A') and at least one of the genetic features (B) to (D), more preferably the genetic feature (A') and all of the genetic features (B) to (D). In an alternative preferable embodiment, the genetic features to be detected are the genetic feature (A') and at least one of the genetic features (B') to (E'), more preferably the genetic feature (A') and all of the genetic features (B') to (E'). In an alternative preferable embodiment, the genetic features to be detected are the genetic feature (A') and at least one of the genetic features (B') to (D'), more preferably the genetic feature (A') and all of the genetic features (B') to (D'). As described above, since the variations related to the genetic features (A) to (E) of the present invention are in linkage disequilibrium, the detection of any one of these genetic features allows the presence or absence of the remaining genetic features to be predicted.

The screening method of the present invention may further comprise a step of selecting from among the test plants a plant in which the presence of at least one genetic feature of the above is detected.

The presence of the genetic feature(s) of the present invention can be determined, for example, by detecting:
(I) the presence of an allele wherein the base at the position corresponding to position 40 of SEQ ID NO: 1 is C (e.g., an allele comprising the nucleotide sequence of SEQ ID NO: 16, 17, 18 or 19; hereinafter, may be referred to as "allele a");
(II) the presence of an allele wherein the base at the position corresponding to position 21 of SEQ ID NO: 2 is T (e.g., an allele comprising the nucleotide sequence of SEQ ID NO: 20, 21, 22 or 23; hereinafter, may be referred to as "allele b");
(III) the presence of an allele wherein the base at the position corresponding to position 28 of SEQ ID NO: 3 is T (e.g., an allele comprising the nucleotide sequence of SEQ ID NO: 24, 25, 26 or 27; hereinafter, may be referred to as "allele c");
(IV) the presence of an allele wherein the base at the position corresponding to position 59 of SEQ ID NO: 4 is C (e.g., an allele comprising the nucleotide sequence of SEQ ID NO: 28, 29, 30 or 31; hereinafter, may be referred to as "allele d"); and/or
(V) the presence of an allele wherein the base at the position corresponding to position 64 of SEQ ID NO: 5 is T (e.g., an allele comprising the nucleotide sequence of SEQ ID NO: 32, 33, 34 or 35; hereinafter, may be referred to as "allele e").

The absence of the genetic feature(s) of the present invention can be determined, for example, by detecting:
(i) the presence of only an allele wherein the base at the position corresponding to position 40 of SEQ ID NO: 1 is T (e.g., an allele comprising the nucleotide sequence of SEQ ID NO: 1, 66, 67 or 68; hereinafter, may be referred to as "allele A");
(ii) the presence of only an allele wherein the base at the position corresponding to position 21 of SEQ ID NO: 2 is A (e.g., an allele comprising the nucleotide sequence of SEQ ID NO: 2, 69, 70 or 71; hereinafter, may be referred to as "allele B");
(iii) the presence of only an allele wherein the base at the position corresponding to position 28 of SEQ ID NO: 3 is C (e.g., an allele comprising the nucleotide sequence of SEQ ID NO: 3, 72, 73 or 74; hereinafter, may be referred to as "allele C");
(iv) the presence of only an allele wherein the base at the position corresponding to position 59 of SEQ ID NO: 4 is A (e.g., an allele comprising the nucleotide sequence of SEQ ID NO: 4, 75, 76 or 77; hereinafter, may be referred to as "allele D"); and/or
(v) the presence of only an allele wherein the base at the position corresponding to position 64 of SEQ ID NO: 5 is C (e.g., an allele comprising the nucleotide sequence of SEQ ID NO: 5, 78, 79 or 80; hereinafter, may be referred to as "allele E").

The presence of the genetic feature (A') of the present invention can be determined, for example, from the presence of only the allele a. The presence of the genetic feature (A") can be determined, for example, from the presence of both the allele a and the allele A.

The presence of the genetic feature (B') of the present invention can be determined, for example, from the presence of only the allele b. The presence of the genetic feature (B") can be determined, for example, from the presence of both the allele b and the allele B.

The presence of the genetic feature (C') of the present invention can be determined, for example, from the presence of only the allele c. The presence of the genetic feature (C") can be determined, for example, from the presence of both the allele c and the allele C.

The presence of the genetic feature (D') of the present invention can be determined, for example, from the presence of only the allele d. The presence of the genetic feature (D") can be determined, for example, from the presence of both the allele d and the allele D.

The presence of the genetic feature (E') of the present invention can be determined, for example, from the presence of only the allele e. The presence of the genetic feature (E") can be determined, for example, from the presence of both the allele e and the allele E.

The absence of the genetic feature (A') of the present invention can be determined, for example, from the presence of the allele A. The absence of the genetic feature (A") can be determined, for example, from the presence of only any one of the allele A and the allele a.

The absence of the genetic feature (B') of the present invention can be determined, for example, from the presence of the allele B. The absence of the genetic feature (B") can be determined, for example, from the presence of only any one of the allele B and the allele b.

The absence of the genetic feature (C') of the present invention can be determined, for example, from the presence of the allele C. The absence of the genetic feature (C") can be determined, for example, from the presence of only any one of the allele C and the allele c.

The absence of the genetic feature (D') of the present invention can be determined, for example, from the presence of the allele D. The absence of the genetic feature (D") can be determined, for example, from the presence of only any one of the allele D and the allele d.

The absence of the genetic feature (E') of the present invention can be determined, for example, from the presence of the allele E. The absence of the genetic feature (E") can be determined, for example, from the presence of only any one of the allele E and the allele e.

Specific examples of methods of detecting the genetic features of the present invention include, but not limited to, PCR method, TaqMan PCR method, sequencing method, microarray method, Invader method, TILLING method, RAD method, RFLP method, PCR-SSCP method, AFLP method, SSLP method, CAPS method, dCAPS method, ASO method, ARMS method, DGGE method, CCM method, DOL method, MALDI-TOF/MS method, TDI method, padlock probe method, molecular beacon method, DASH method, UCAN method, ECA method, PINPOINT method, PROBE method, VSET method, Survivor assay, Sniper assay, Luminex assay, GOOD method, LCx method, SNaPshot method, Mass ARRAY method, pyrosequencing method, SNP-IT method, melting curve analysis method, etc.

In the case of PCR method, it is preferable to generate a primer such that the 3' end portion has a sequence complementary to the variation site of the present invention. By using a primer designed in this way, the polymerase extension reaction proceeds because the primer hybridizes completely to the template if the template sample has the variation of the present invention, whereas if the template does not have the variation of the present invention, the extension reaction does not occur because the nucleotide at the 3' end of the primer mismatches the template. Therefore, PCR amplification is performed using such a primer, and the amplification product is analyzed by agarose gel electrophoresis or the like, and if an amplification product of a predetermined size can be confirmed, the template as the sample has a variation, and if the amplification product is not present, it can be judged that the template does not have a variation.

Alternatively, the genetic feature(s) of the present invention can be detected by designing the primer sequence so that the variation of the present invention and the primer sequence do not overlap and the genetic variation of the present invention can be PCR amplified, and by sequencing the nucleotide sequence of the amplified nucleotide fragment.

For PCR and agarose gel electrophoresis see Sambrook, Fritsch and Maniatis, "Molecular Cloning: A Laboratory Manual" 2nd Edition (1989), Cold Spring Harbor Laboratory Press.

TaqMan PCR method uses fluorescently labeled allele-specific oligos and Taq DNA polymerases (Livak, K. J. Genet. Anal. 14, 143 (1999); Morris T. et al., J. Clin. Microbiol. 34, 2933 (1996)).

The sequencing method is a method of analyzing the presence or absence of a variation by amplifying a region containing the variation by PCR and sequencing the DNA sequence using a Dye Terminator or the like (Sambrook, Fritsch and Maniatis, "Molecular Cloning: A Laboratory Manual" 2nd Edition (1989), Cold Spring Harbor Laboratory Press).

A DNA microarray is one in which one end of a nucleotide probe is immobilized in an array on a support, and includes a DNA chip, a Gene chip, a microchip, a bead array, and the like. By using a probe containing a sequence complementary to the variation site of the present invention, the presence or absence of the polymorphism of the present invention can be comprehensively detected. DNA microarray assays such as DNA chips include GeneChip assays (see Affymetrix; U.S. Pat. Nos. 6,045,996; 5,925,525; and 5,858,659). The GeneChip technique utilizes a miniaturized, high density microarray of oligonucleotide probes affixed to a chip.

The invader method combines the hybridization of two reporter probes specific for each allele of a variation such as SNPs and one invader probe to template DNA and the cleavage of DNA by Cleavase enzyme with a special endonuclease activity which cleaves a DNA by recognizing its structure (Livak, K. J. Biomol. Eng. 14, 143-149 (1999); Morris T. et al., J. Clin. Microbiol. 34, 2933 (1996); Lyamichev, V. et al., Science, 260, 778-783 (1993), and the like).

TILLING (Targeting Induced Local Lesions IN Genomes) method is a method in which mutational mismatches in the genomes of a mutagenized mutant population are screened by PCR-amplification and CEL I nuclease-treatment.

In one embodiment, the genetic feature (A) of the present invention can be detected, for example, by dCAPS method using the following primer set and a restriction enzyme.

### Primer set:

A primer set comprising a forward primer comprising a continuous sequence of 15 to 39-base long from the 3' end of SEQ ID NO: 36 and a reverse primer comprising a sequence (e.g., SEQ ID NO: 37) complementary to any continuous sequence of 15 bases or more which is positioned at the 3' side of position 41 of SEQ ID NO: 1 or 16, or a primer set comprising a forward primer comprising a continuous sequence of 15 to 20-base long from the 3' end of SEQ ID NO: 81 or 82 and a reverse primer comprising a sequence (e.g., SEQ ID NO: 85) complementary to any continuous sequence of 15 bases or more which is positioned at the 3' side of position 22 of SEQ ID NO: 83 or 84.

### Restriction enzyme:

A restriction enzyme for the primer set based on SEQ ID NO: 36 includes RsaI, a restriction enzyme for the primer set based on SEQ ID NO: 81 includes Tsp451, and a restriction enzyme for the primer set based on SEQ ID NO: 82 includes AciI.

In one embodiment, the genetic feature (B) of the present invention can be detected, for example, by dCAPS method using the following primer set and a restriction enzyme.

### Primer set:

A primer set comprising a forward primer comprising a continuous sequence of 15 to 20-base long from the 3' end of SEQ ID NO: 42, 86 or 87 and a reverse primer comprising a sequence (e.g., SEQ ID NO: 43) complementary to any continuous sequence of 15 bases or more which is positioned at the 3' side of position 22 of SEQ ID NO: 2 or 20.

### Restriction enzyme:

A restriction enzyme for the primer set based on SEQ ID NO: 42 includes AclI, a restriction enzyme for the primer set based on SEQ ID NO: 86 includes AluI, and a restriction enzyme for the primer set based on SEQ ID NO: 87 includes Pad.

In one embodiment, the genetic feature (C) of the present invention can be detected, for example, by dCAPS method using the following primer set and a restriction enzyme.

### Primer set:

A primer set comprising a forward primer comprising a continuous sequence of 15 to 20-base long from the 3' end of SEQ ID NO: 48, 88 or 89 and a reverse primer comprising a sequence (e.g., SEQ ID NO: 49) complementary to any continuous sequence of 15 bases or more which is positioned at the 3' side of position 22 of SEQ ID NO: 50 or 51.

### Restriction enzyme:

A restriction enzyme for the primer set based on SEQ ID NO: 48 includes AluI, a restriction enzyme for the primer set based on SEQ ID NO: 88 includes BglI, and a restriction enzyme for the primer set based on SEQ ID NO: 89 includes Seal.

In one embodiment, the genetic feature (D) of the present invention can be detected, for example, by dCAPS method using the following primer set and a restriction enzyme.

### Primer set:

A primer set comprising a forward primer comprising a continuous sequence of 15 to 21-base long from the 3' end of SEQ ID NO: 54, 90 or 91 and a reverse primer comprising a sequence (e.g., SEQ ID NO: 55) complementary to any continuous sequence of 15 bases or more which is positioned at the 3' side of position 23 of SEQ ID NO: 56 or 57.

### Restriction enzyme:

A restriction enzyme for the primer set based on SEQ ID NO: 54 includes AluI, a restriction enzyme for the primer set based on SEQ ID NO: 90 includes MboI, and a restriction enzyme for the primer set based on SEQ ID NO: 91 includes BglII.

In one embodiment, the genetic feature (E) of the present invention can be detected, for example, by dCAPS method using the following primer set and a restriction enzyme.

### Primer set:

A primer set comprising a forward primer comprising a continuous sequence of 15 to 22-base long from the 3' end of SEQ ID NO: 60, 92 or 93 and a reverse primer comprising a sequence (e.g., SEQ ID NO: 61) complementary to any continuous sequence of 15 bases or more which is positioned at the 3' side of position 24 of SEQ ID NO: 62 or 63.

### Restriction enzyme:

A restriction enzyme for the primer set based on SEQ ID NO: 60 includes AluI, a restriction enzyme for the primer set based on SEQ ID NO: 92 includes MboI, and a restriction enzyme for the primer set based on SEQ ID NO: 93 includes BglII.

The sequences of the primers can be optimized within a range that satisfies the conditions described above. For the optimization of primer design, see, for example, Sambrook and Russell, "Molecular Cloning: A Laboratory Manual" 3rd Edition (2001), Cold Spring Harbor Laboratory Press. Each of the primers may be 15 to 50-base long, 18 to 48-base long, 20 to 45-base long, 30 to 40-base long, or the like. The restriction enzyme for each primer set also includes other enzymes that recognize the same sequence and cleave the same site as in the above enzyme, or an isoschizomer of the above enzyme. It is also possible to design a primer set other than those described above on the basis of the genetic feature(s) of the present invention and select a restriction enzyme appropriate therefor.

In a specific embodiment, the genetic features (A) to (E) of the present invention can be detected, e.g., by dCAPS method using the primer set having the following sequence and restriction enzyme.

**Table 1 Examples of combination of primer set and restriction enzyme for detecting genetic feature**

| (A) | | |
|---|---|---|
| Forward primer | Reverse primer | Restriction enzyme |
| SEQ ID NO: 36 | SEQ ID NO: 37 | RsaI |
| SEQ ID NO: 81 | SEQ ID NO: 85 | Tsp451 |
| SEQ ID NO: 82 | SEQ ID NO: 85 | AciI |

**Table 2 Examples of combination of primer set and restriction enzyme for detecting genetic feature**

| (B) | | |
|---|---|---|
| Forward primer | Reverse primer | Restriction enzyme |
| SEQ ID NO: 42 | SEQ ID NO: 43 | AclI |
| SEQ ID NO: 86 | SEQ ID NO: 43 | AluI |
| SEQ ID NO: 87 | SEQ ID NO: 43 | PacI |

**Table 3 Examples of combination of primer set and restriction enzyme for detecting genetic feature**

| (C) | | |
|---|---|---|
| Forward primer | Reverse primer | Restriction enzyme |
| SEQ ID NO: 48 | SEQ ID NO: 49 | AluI |
| SEQ ID NO: 88 | SEQ ID NO: 49 | BglI |
| SEQ ID NO: 89 | SEQ ID NO: 49 | Seal |

**Table 4 Examples of combination of primer set and restriction enzyme for detecting genetic feature**

| (D) | | |
|---|---|---|
| Forward primer | Reverse primer | Restriction enzyme |
| SEQ ID NO: 54 | SEQ ID NO: 55 | AluI |
| SEQ ID NO: 90 | SEQ ID NO: 55 | MboI |
| SEQ ID NO: 91 | SEQ ID NO: 55 | BglII |

**Table 5 Examples of combination of primer set and restriction enzyme for detecting genetic feature**

| (E) | | |
|---|---|---|
| Forward primer | Reverse primer | Restriction enzyme |
| SEQ ID NO: 60 | SEQ ID NO: 61 | AluI |
| SEQ ID NO: 92 | SEQ ID NO: 61 | MboI |
| SEQ ID NO: 93 | SEQ ID NO: 61 | BglII |

The combinations of the primer set and the restriction enzyme described above are mere examples, and other combinations of primer sets and restriction enzymes capable of detecting the genetic feature(s) of the present invention can be found by those skilled in the art.

The screening methods of the present invention may further comprise a step of determining the content of RebE of a tissue (e.g., a leave) of the test stevia plant. The determination of the content of RebE is as described in the section relating to the plant of the present invention. In this embodiment, the screening method of the present invention may be applied to daughter plants obtained by selecting individuals with a higher content of RebE from among the test stevia plants in which the genetic feature(s) of the present invention is/are detected, and crossing the selected individuals with another stevia plants. Thus, the screening method of the present invention comprises a step of detecting from the genome of a test stevia plant the presence and/or the absence of the genetic feature(s) of the present inventions, and may further comprise one or more of the following steps.
(i) Determining the content of RebE of the test stevia plant tissue (e.g., a leaf) in which the genetic feature(s) of the present invention has/have been detected;
(ii) selecting an individual with a higher content of RebE from among the test stevia plants in which the genetic feature(s) of the present invention has/have been detected;
(iii) crossing the selected individual with a higher content of RebE with another stevia plant;
(iv) detecting the genetic feature(s) of the present invention from the genome of daughter plants obtained by crossing,
(v) measuring the content of RebE of the tissue of the daughter plants in which the genetic feature(s) of the present invention has/have been detected,
(vi) selecting individuals having a higher content of RebE from among the daughter plants in which the genetic features are detected.

The screening method of the present invention may comprise one or more of the following steps.
(i') Determining the content of stevioside or TSG of the test stevia plant tissue (e.g., a leaf) in which the genetic feature(s) of the present invention has/have been detected;
(ii') selecting an individual with a higher RebE/stevioside or a higher mass ratio of RebE to TSG from among the test stevia plants in which the genetic feature(s) of the present invention has/have been detected;
(iii') crossing the selected individual with a higher RebE/stevioside or a higher mass ratio of RebE to TSG with another stevia plant;
(iv') detecting the genetic feature(s) of the present invention from the genome of daughter plants obtained by crossing,
(v') measuring the content of stevioside or TSG of the tissue of the daughter plants in which the genetic feature(s) of the present invention has/have been detected,
(vi') selecting individuals having a higher RebE/stevioside or a higher mass ratio of RebE to TSG from among the daughter plants in which the genetic features of the present invention are detected.

Individuals with a high content of RebE, a high RebE/stevioside or a high mass ratio of RebE to TSG of choice may be, for example, up to 50%, up to 40%, up to 30%, up to 20%, up to 10%, up to 5%, up to 4%, up to 3%, up to 2%, or up to 1% of the test stevia plants in which the genetic feature(s) of the present invention has/have been detected, with respect to the level of the content of RebE, the level of RebE/stevioside or the level of the mass ratio of RebE to TSG. Other stevia plants to be crossed may or may not contain the genetic feature(s) of the present invention. In the above embodiment, steps (iii) to (vi) or steps (iii') to (vi') can be repeated a plurality of times. In this way, stevia plants with a higher content of RebE, a higher RebE/stevioside or a higher mass ratio of RebE to TSG can be screened.

In the screening method of the present invention, the test stevia plant may be a natural stevia plant or a non-genetically modified stevia plant. Non-genetically modified stevia plants are as described in the section relating to the plant of the present invention.

In the screening method of the present invention, the test stevia plant may include a stevia plant subjected to a mutagenesis treatment and a progeny plant thereof. The induction of a variation is as described in the section relating to the plant of the present invention, and includes treatment with a mutagen, treatment with radiation or irradiation with light, and the like.

The amount of RebE, the amount of stevioside, the amount of RebE with respect to the amount of TSG, etc. in the plant obtained by the screening method of the present invention are as described in the section relating to the plant of the present invention.

The present invention also provides the primer sets described above and combinations thereof, for example, the primer sets described above in Tables 1 to 5 and combinations of these primer sets. The present invention further provides a primer set capable of amplifying a region having a nucleotide sequence selected from the group consisting of SEQ ID NOs: 1 to 5, 16, 20, 24, 28 and 32 by PCR, for example, a primer set with a forward primer comprising a nucleotide sequence of SEQ ID NO: 6, and a reverse primer comprising a nucleotide sequence of SEQ ID NO: 7; a primer set with a forward primer comprising a nucleotide sequence of SEQ ID NO: 8, and a reverse primer comprising a nucleotide sequence of SEQ ID NO: 9; a primer set with a forward primer comprising a nucleotide sequence of SEQ ID NO: 10, and a reverse primer comprising a nucleotide sequence of SEQ ID NO: 11; a primer set with a forward primer comprising a nucleotide sequence of SEQ ID NO: 12, and a reverse primer comprising a nucleotide sequence of SEQ ID NO: 13; and a primer set with a forward primer comprising a nucleotide sequence of SEQ ID NO: 14, and a reverse primer comprising a nucleotide sequence of SEQ ID NO: 15; and the like.

In addition, the present invention provides a probe capable of detecting the presence and/or absence of the genetic features of the present invention, which may be referred to as the "probe of the present invention" hereinafter. The probe of the present invention may have a structure suitable for various detection methods (e.g., realtime PCR method such as TaqMan PCR method and the like) for the presence and/or absence of the genetic feature(s) of the present invention. For example, the probe of the present invention may comprise a nucleotide sequence complementary to a portion of a genome comprising a variation site of the present invention. Non-limiting examples of such probes include those comprising a sequence complementary to a nucleotide sequence selected from SEQ ID NOs: 17 to 19, 21 to 23, 25 to 27, 29 to 31, 33 to 35 and 66 to 80. Of these sequences, SEQ ID NOs: 17 to 19, 21 to 23, 25 to 27, 29 to 31 and 33 to 35 are specific for alleles comprising the variation of the present invention, and SEQ ID NOs: 66 to 80 are specific for alleles not containing the variation of the present invention. Further, SEQ ID NOs: 17 to 19 are specific for allele a, SEQ ID NOs: 21 to 23 are specific for allele b, SEQ ID NOs: 25 to 27 are specific for allele c, SEQ ID NOs: 29 to 31 are specific for allele d, and SEQ ID NOs: 33 to 35 are specific for allele e. On the other hand, SEQ ID NOs: 66 to 68 are specific for allele A, SEQ ID NOs: 69 to 71 are specific for allele B, SEQ ID NOs: 72 to 74 are specific for allele C, SEQ ID NOs: 75 to 77 are specific for allele D, and SEQ ID NOs: 78 to 80 are specific for allele E The presence of the genetic features (A) to (E) of the present invention may be detected by detection of only an allele comprising the variation of the present invention, or, of both of an allele comprising the variation of the present invention and an allele not comprising the variation of the present invention, and the absence of the genetic features (A) to (E) of the present invention by detection of only an allele not comprising the variation of the present invention. The presence of the genetic features (A') to (E') of the present invention may be detected by detection of only an allele comprising the variation of the present invention, and the absence of the genetic feature (A') to (E') of the present invention by detection of an allele not comprising the variation of the present invention. The probes of the present invention preferably have a label. Non-limiting examples of such labels include fluorescent labels, luminescent labels, radioactive labels, dyes, enzymes, quenchers, binding moieties with detectable labels, and the like. In a specific embodiment, the probe of the present invention has a polynucleotide comprising a nucleotide sequence complementary to a nucleotide sequence selected from SEQ ID NOs: 17 to 19, 21 to 23, 25 to 27, 29 to 31, 33 to 35 and 66 to 80, and a label.

The present invention further provides a kit comprising the above-mentioned primer set and a restriction enzyme appropriate therefor. In a specific embodiment, the kit of the present invention comprises a primer set stated in the above Tables 1-5 and a restriction enzyme appropriate therefor.

The present invention also provides a kit comprising a primer set capable of amplifying by PCR a region having a nucleotide sequence selected from the group consisting of SEQ ID NOs: 1 to 5, 16, 20, 24, 28 and 32, and the above-mentioned probe of the present invention appropriate therefor.

These primer sets, probes and kits can be used to detect the genetic feature(s) of the present invention, used in the screening methods of the present invention, and the like. These primer sets and kits may also comprise an instruction including an explanation on the detection of genetic feature(s) of the present invention and on the screening method of the present invention, e.g., a written instruction, information of a site comprising information regarding the method of use (e.g., URL and 2D code), and media, e.g., a flexible disk, a CD, a DVD, a Blu-ray disk, a memory card, a USB memory, etc., having recorded thereon information regarding the method of use, and the like.

In some embodiments, the present invention provides a screening kit for the stevia plant of the present invention, comprising a reagent for detecting the presence and/or the absence of at least one of the genetic features (A) to (E). The reagent may comprise a primer and/or a probe for use in CAPS method, dCAPS method or TaqMan PCR method. In a specific embodiment, the reagent for detecting the presence and/or the absence of at least one of the genetic features (A) to (E) comprises a combination of a primer set and a restriction enzyme for detecting at least one of the above genetic features (A) to (E) by the dCAPS method, for instance a combination of a primer set and a restriction enzyme stated in Tables 1-5, or a combination of a primer set that amplifies the variation site(s) of the present invention (e.g., a site comprising a sequence selected from SEQ ID NOs: 16 to 35), and a probe having a nucleotide sequence complementary to a site related to at least one of the genetic features (A) to (E) (e.g., a site comprising a sequence selected from SEQ ID NOs: 17 to 19, 21 to 23, 25 to 27, 29 to 31 and 33 to 35), which can be used in the TaqMan PCR method or the like.

### 4. Method of producing RebE-containing extract derived from plant, the extract, and product comprising the extract

In a further aspect, the present invention provides a method of producing a RebE-containing extract, comprising a step of obtaining a RebE-containing extract from the plant of the present invention, a stevia plant selected by the screening method of the present invention or a stevia plant produced by the production method of the present invention, or a seed, a leaf (e.g., dried leaf or fresh leaf), a tissue, a tissue culture or a cell of the plant (hereinafter, may be referred to as the "RebE-containing extract production method of the present invention").

Further provided is a RebE-containing extract from the plant of the present invention, a stevia plant selected by the screening method of the present invention or a stevia plant produced by the production method of the present invention, or a seed, a leaf (e.g., dried leaf or fresh leaf), a tissue, a tissue culture or a cell of the plant (hereinafter, may be referred to as the "RebE-containing extract of the present invention"). The RebE-containing extract of the present invention is preferably produced by the RebE-containing extract production method of the present invention. Furthermore provided is a method of producing RebE, comprising a step of purifying RebE from the RebE-containing extract of the present invention (hereinafter, may be referred to as the "RebE production method of the present invention"). The RebE production method of the present invention may further comprise a step of obtaining a RebE-containing extract from the plant of the present invention, a stevia plant selected by the screening method of the present invention or a stevia plant produced by the production method of the present invention.

The RebE-containing extract can be obtained by reacting a fresh leaf or a dried leaf of the plant of the present invention with a suitable solvent (an aqueous solvent such as water or an organic solvent such as an alcohol, ether or acetone). For the extraction conditions, etc., see a method described in Ohta et al., supra or WO2010/038911, or a method described in Examples mentioned later.

The RebE-containing extract can be purified by use of a method known in the art such as a gradient of ethyl acetate or any of other organic solvents:water, high performance liquid chromatography (HPLC), gas chromatography, time-of-flight mass spectrometry (TOF-MS), or ultra (high) performance liquid chromatography (UPLC).

The RebE-containing extract of the present invention comprises RebE at higher content as compared with a RebE-containing extract obtained from a stevia species not having the genetic feature of the present invention.

The RebE-containing extract of the present invention may comprise RebE at higher content by more than 100 %, 105 % or more, 109 % or more, 150 % or more, 200 % or more, 210 % or more, 290 % or more, 400 % or more, 600 % or more, 800 % or more, 1000 % or more, 1200 % or more, 1300 % or more, 1400 % or more, 1500 % or more, 1600 % or more, 1700 % or more, 1800 % or more, 1900 % or more, 2000 % or more, 2100 % or more, 2200 % or more, 2300 % or more, 2400 % or more, 2500 % or more, 2600 % or more, 2700 % or more, 2800 % or more, 2900 % or more, 3000 % or more, 3100 % or more, 3200 % or more, 3300 % or more, 3400 % or more, 3500 % or more, 3600 % or more, 3700 % or more, 3800 % or more, 3900 % or more, 4000 % or more, 4100 % or more, 4200 % or more, 4300 % or more, 4400 % or more, 4500 % or more, 4600 % or more, 4700 % or more, 4800 % or more, 4900 % or more, 5000 % or more as compared with a RebE-containing extract obtained from a stevia species not having the genetic feature of the present invention. The upper limit is not particularly limited and may be, for example, 21000 % or less, 18000 % or less, or the like. The RebE-containing extract of the present invention and the RebE-containing extract obtained from the stevia species not having the genetic feature of the present invention may be those obtained by the same process.

The RebE-containing extract of the present invention thus obtained and/or RebE obtained by the RebE production method of the present invention can be mixed with other component(s) to produce a novel food or beverage, sweetener composition, flavor or medicament with increased content of RebE. Accordingly, in an alternative aspect, the present invention provides a method of producing a food or beverage, a sweetener composition, a flavor or a medicament, comprising a step of providing the RebE-containing extract of the present invention, and/or RebE obtained by the RebE production method of the present invention, and a step of adding the extract and/or RebE to a raw material for the food or beverage, sweetener composition, flavor or medicament.

The present invention further provides a novel food or beverage, sweetener composition, flavor or medicament with increased content of RebE, obtained by the production method. In this context, the food or beverage means a beverage and a food. Thus, in a certain embodiment, the present invention provides a novel beverage, food, sweetener composition, flavor or medicament and also provides a method of producing the beverage, food, sweetener composition, flavor or medicament.

### 5. Nucleotide sequence relating to plant of present invention

In another aspect, the present invention provides nucleotide sequences relating to the plant of the present invention.

Nucleotide sequences relating to a stevia plant having the genetic feature (A) comprise or consist of a nucleotide sequence selected from SEQ ID NOs: 16 to 19. Nucleotide sequences relating to a stevia plant having the genetic feature (B) comprise or consist of a nucleotide sequence selected from SEQ ID NOs: 20 to 23. Nucleotide sequences relating to a stevia plant having the genetic feature (C) comprise or consist of a nucleotide sequence selected from SEQ ID NOs: 24 to 27. Nucleotide sequences relating to a stevia plant having the genetic feature (D) comprise or consist of a nucleotide sequence selected from SEQ ID NOs: 28 to 31. Nucleotide sequences relating to a stevia plant having the genetic feature (E) comprise or consist of a nucleotide sequence selected from SEQ ID NOs: 32 to 35.

### EXAMPLES

Hereinafter, the present invention will be described with reference to Examples, etc. However, the present invention is not limited by these specific embodiments.

### Example 1: Preparation of stevia plant with high RebE content

The present inventors repeated the selection and crossing of stevia plants in order to obtain stevia plants rich in RebD. During this process, the present inventors realized the presence of stevia plants rich in RebE. Specific procedures are as follows.

### 1. Production of test lines

A wild type stevia species (commercially available variety) was treated with ethyl methanesulfonate (EMS), and the resultant was seeded and cultivated in a greenhouse within the Suntory World Research Center. An appropriate amount of fresh leaves was sampled from each grown individual, and the concentration of RebD was quantitatively determined by LC-MS/MS (Shimadzu LCMS8050). Specifically, 0.25 g of the fresh leaves was dried by freeze drying, and 0.05 g of homogenized dry matter thereof was added into a 100-fold amount (5 mL) of pure water. Extraction by ultrasonic treatment for 20 minutes, and centrifugation and filtration were performed, followed by 60-fold dilution with 32% acetonitrile to obtain a liquid sample. The concentration of RebD was quantitatively determined by LC/MS-MS analysis on this 1 mL of liquid sample in a LCMS8050 MRM mode, and individuals having the concentration of 2% or more were selected and crossed to obtain seeds. Such selection was repeated over four generations to obtain Populations A and B of the fourth filial generation (S4 generation).

### 2. Measurement of steviol glycoside contained in each individual

An appropriate amount of fresh leaves was sampled from each individual of Populations A and B. The concentrations (% by mass with respect to a dried leaf) of RebA, RebB, RebC, RebD, RebE, RebF, RebG, RebM, RebN and stevioside (STV) were quantitatively determined by LC/MS-MS analysis in the same way as in the preceding section 1, and the total sum thereof was regarded as the concentration of TSG. The results are shown in the table below. Each value in the table is a measurement value of one individual of the stevia plant.

**Table 6 Glycoside concentration in Population A**

| **Line number** | **% per 100** g | **of dried** | **leaf** | **% per** | **TSG** | **RebE/ Stevioside** |
|---|---|---|---|---|---|---|
| | Stevioside | RebE | TSG | Stevioside | RebE | |
| S41-1 | 4.9 | 4.16 | 9.1 | 53.7 | 45.8 | 0.85 |
| S41-2 | 5.5 | 4.11 | 9.6 | 56.6 | 42.7 | 0.75 |
| S41-3 | 5.3 | 3.47 | 8.9 | 60.2 | 391 | 0.65 |
| S41-4 | 5.7 | 3.46 | 92 | 61.7 | 37.4 | 0.61 |
| S41-5 | 50 | 3.40 | 8.5 | 592 | 400 | 0.68 |
| S41-6 | 4.7 | 3.39 | 8.2 | 57.7 | 41.5 | 0.72 |
| S41-7 | 62 | 324 | 96 | 65.2 | 339 | 0.52 |
| S41-8 | 6.8 | 3.15 | 10.0 | 67.9 | 31.5 | 0.46 |
| S41-9 | 7.6 | 3.06 | 10.7 | 70.7 | 286 | 0.40 |
| S41-10 | 5.4 | 2.98 | 8.5 | 64.3 | 352 | 0.55 |
| S41-11 | 5.4 | 2.97 | 84 | 642 | 35.2 | 0.55 |
| S41-12 | 53 | 294 | 82 | 636 | 355 | 056 |
| S41-13 | 5.8 | 2.91 | 8.7 | 65.8 | 333 | 0.51 |
| S41-14 | 49 | 2.90 | 8.0 | 61.6 | 365 | 0.59 |
| S41-15 | 4.6 | 2.90 | 7 5 | 61.0 | 38.5 | 0.63 |
| S41-16 | 5.2 | 2.75 | 80 | 64.7 | 34.3 | 0.53 |
| S41-17 | 45 | 2.73 | 7.3 | 61.9 | 37.6 | 0.61 |
| S41-18 | 4.1 | 230 | 6.4 | 635 | 35.8 | 0.55 |
| S41-19 | 56 | 225 | 79 | 709 | 285 | 0.40 |
| S41-20 | 6.5 | 2.24 | 8.8 | 73.6 | 25.5 | 0.35 |
| S41-21 | 5.3 | 211 | 7.5 | 71.1 | 281 | 040 |
| S41-22 | 4.1 | 2.10 | 6.3 | 65.4 | 33.5 | 0.51 |
| S41-23 | 2.0 | 0.62 | 7.0 | 28.7 | 8.9 | 0.31 |
| S41-24 | 2.6 | 0.58 | 9.0 | 28.7 | 65 | 0.23 |
| S41-25 | 2.3 | 0.55 | 10.0 | 22.9 | 5.6 | 0.24 |
| S41-26 | 2.1 | 0.55 | 8.4 | 24.8 | 6.5 | 0.26 |
| S41-27 | 2.6 | 0.54 | 8.2 | 31.7 | 66 | 0.21 |
| S41-28 | 24 | 053 | 8.5 | 280 | 62 | 0.22 |
| S41-29 | 2.5 | 0.49 | 8.7 | 28.2 | 5.6 | 0.20 |
| S41-30 | 1.9 | 0.45 | 7.8 | 24.4 | 5.7 | 0.22 |
| S41-31 | 1.5 | 0.43 | 8.7 | 17.2 | 5.0 | 0.29 |
| S41-32 | 17 | 0.43 | 5.9 | 29.0 | 73 | 026 |
| S41-33 | 21 | 0.41 | 6.8 | 30.4 | 6.0 | 0.20 |
| S41-34 | 1.5 | 0.40 | 7.0 | 21.6 | 5.7 | 0.26 |
| S41-35 | 27 | 040 | 91 | 29.6 | 43 | 0.15 |
| S41-36 | 21 | 0.39 | 7.9 | 26.6 | 49 | 0.19 |
| S41-37 | 1.6 | 0.39 | 7.1 | 22.4 | 55 | 0.25 |
| S41-38 | 1.7 | 0.39 | 8.6 | 20.2 | 45 | 022 |
| S41-39 | 20 | 0.35 | 7.9 | 258 | 45 | 0.18 |
| S41-40 | 1.6 | 0.36 | 5.9 | 27.8 | 6.1 | 0.22 |
| S41-41 | 2.1 | 0.36 | 8.1 | 26.3 | 4.4 | 0.17 |
| S41-42 | 1.5 | 0.35 | 6.1 | 25.0 | 58 | 0.23 |
| S41-43 | 1.5 | 0.34 | 7.5 | 20.4 | 4.6 | 0.23 |
| S41-44 | 1.7 | | 6.4 | 266 | 54 | 0.20 |
| S41-45 | 1.5 | 0.33 | 5.8 | 255 | 57 | 0.22 |
| S41-46 | 22 | 0.32 | 11.0 | 20.1 | 29 | 0.15 |
| S41-47 | 1.5 | 0.32 | 7.7 | 19.1 | 42 | 0.22 |
| S41-48 | 1.6 | 0.32 | 6.8 | 23.9 | 47 | 0.20 |
| S41-49 | 2.1 | 032 | 76 | 27.8 | 41 | 0.15 |
| S41-50 | 1.3 | 0.31 | 5.5 | 24.5 | 5.t | 0.23 |
| S41-51 | 1.5 | 0.30 | 7.0 | 1.7 | 43 | 0.20 |
| S41-52 | 1.4 | 0.30 | 7.2 | 19.1 | 42 | 0.22 |
| S41-53 | 1.2 | 0.30 | 7.1 | 16.6 | 42 | 025 |
| S41-54 | 1.4 | 0.30 | 7.6 | 17.9 | 39 | 0.22 |
| S41-55 | 15 | 0.29 | 8.1 | 187 | 36 | 0.19 |
| S41-56 | 2.0 | 0.28 | 8.9 | 23.0 | 32 | 0.14 |
| S41-57 | 2 5 | 0.27 | 10.1 | 250 | 26 | 0.11 |
| S41-58 | 2.1 | 0.26 | 97 | 21.6 | 2.6 | 0.12 |
| S41-59 | 1.2 | 0.25 | 6.5 | 18.2 | 3.9 | 0.21 |
| S41-60 | 2.2 | 0.25 | 9.8 | 22.0 | 25 | 0.11 |
| S41-61 | 1.8 | 0.25 | 7.1 | 25.0 | 35 | 0.14 |
| S41-62 | 15 | 0.22 | 7.4 | 209 | 31 | 0.15 |
| S41-63 | 1.0 | 0.22 | 6.5 | 15.5 | 33 | 0.22 |
| S41-64 | 1.3 | 0.21 | 7.2 | 17.6 | 30 | 0.17 |
| S41-65 | 1.3 | 0.21 | 73 | 18.3 | 2.8 | 0.16 |
| S41-66 | 1.8 | 0.20 | 6.8 | 27.3 | 29 | 0.11 |
| S41-67 | 1.1 | 0.20 | 6.7 | 161 | 29 | 0.16 |
| S41-68 | 1.8 | 0.19 | 6.3 | 27.9 | 3.1 | 0.11 |
| S41-69 | 14 | 0.18 | 78 | 179 | 24 | 013 |
| S41-70 | 1.5 | 0.18 | 7.1 | 21.4 | 25 | 0.12 |
| S41-71 | 14 | 0.18 | 74 | 18.7 | 24 | 0.13 |
| S41-72 | 1.2 | 0.17 | 6.5 | 18.3 | 2.6 | 0.14 |
| S41-73 | 12 | 0.15 | 60 | 19.4 | 24 | 0.13 |
| S41-74 | 0,6 | 0.10 | 6.0 | 10.0 | 17 | 0.17 |
| S41-75 | 0.7 | 0.09 | 6.5 | 11.0 | 15 | 0.13 |
| S41-76 | 0.6 | 0.08 | 4.8 | 128 | 17 | 0.13 |
| S41-77 | 0.7 | 0.07 | 76 | 9.6 | 1.0 | 0.10 |
| S41-78 | 0.5 | 007 | 8.0 | 57 | 0.8 | 014 |
| S41-79 | 0.5 | 0.06 | 7.1 | 7.5 | 09 | 0.12 |
| S41-80 | 0.7 | 0.06 | 6.4 | 108 | 09 | 0.08 |
| S41-81 | 0.8 | 0.05 | 5.9 | 13.5 | 09 | 0.07 |
| S41-82 | 06 | 0.05 | 94 | 60 | 06 | 009 |
| S41-83 | 0.6 | 0.05 | 8.5 | 7.0 | 05 | 0.08 |
| S41-84 | 0.6 | 0.05 | 8.7 | 6.5 | 05 | 0.08 |
| S41-85 | 07 | 0.04 | 52 | 141 | 09 | 0.06 |
| S41-86 | 0.3 | 0.04 | 6.8 | 4.5 | 0.6 | 0.14 |
| S41-87 | 0.7 | 0.04 | 7.2 | 99 | 06 | 0.06 |
| S41-88 | 0.5 | 0.04 | 8.0 | 6.5 | 05 | 0.08 |
| S41-89 | 0.4 | 0.04 | 54 | 80 | 08 | 0.10 |
| S41-90 | 0.5 | 0.04 | 5.9 | 8.0 | 0.6 | 0.08 |
| S41-91 | 0.5 | 0.03 | 7.7 | 6.8 | 0.4 | 0.06 |
| S41-92 | 0.5 | 0.03 | 7.2 | 6.5 | 04 | 0.06 |
| S41-93 | 0.5 | 0.03 | 6.4 | 71 | 05 | 0.06 |
| S41-94 | 04 | 0.03 | 74 | 57 | 03 | 0.06 |
| S41-95 | 0.5 | 0.02 | 1.4 | 7.4 | 03 | 0.05 |
| S41-96 | 0.5 | 0.02 | 64 | 7.9 | 03 | 0.04 |

As shown in the results, S41-1 to S41-73 in Population A contained RebE in an amount exceeding 0.10% by mass based on a dried leaf, i.e., were rich in RebE. Among them, S41-1 to S41-22 contained RebE in an amount exceeding 0.62% by mass based on a dried leaf, i.e., were particularly rich in RebE.

**Table 7 Glycoside concentration in Population B**

| **Line number** | **% per 100 g of dried leaf** | | | **% per TSG** | | **RebE/ Stevioside** |
|---|---|---|---|---|---|---|
| | Steviosid | RebE | TSG | Stevioside | RebE | |
| S42-1 | 7.3 | 3.52 | 10.9 | 67.5 | 324 | 0.48 |
| S42-2 | 5.3 | 341 | 87 | 60.6 | 393 | 0.65 |
| S42-3 | 7.3 | 3.38 | 107 | 681 | 31.7 | 047 |
| S42-4 | 7.3 | 3.36 | 10.7 | 68.5 | 315 | 0.46 |
| S42-5 | 6.8 | 331 | 10.1 | 61.0 | 329 | 0.49 |
| S42-6 | 5.0 | 3.26 | 8.3 | 60.6 | 393 | 0.65 |
| S42-7 | 6.2 | 325 | 95 | 65.5 | 343 | 0.52 |
| S42-8 | 7.1 | 325 | 104 | 68.7 | 31.2 | 0.45 |
| S42-9 | 8.0 | 3.15 | 11.1 | 71.6 | 283 | 0.40 |
| S42-10 | 7.5 | 3.02 | 105 | 71.2 | 287 | 040 |
| S42-11 | 8.1 | 2.95 | 11.0 | 73.1 | 268 | 0.37 |
| S42-12 | 5.9 | 2.94 | 89 | 66.8 | 331 | 0.50 |
| S42-13 | 6.1 | 2.89 | 90 | 678 | 321 | 047 |
| S42-14 | 6.7 | 287 | 9.5 | 69.8 | 30.1 | 0.43 |
| S42-15 | 6.0 | 279 | 88 | 68.3 | 316 | 046 |
| S42-16 | 6.7 | 2.74 | 9.4 | 70.8 | 291 | 0.41 |
| S42-17 | 7.4 | 271 | 10.2 | 73.3 | 267 | 0.36 |
| S42-18 | 7.7 | 2.68 | 104 | 74.1 | 258 | 0.35 |
| S42-19 | 6.8 | 2.47 | 9.2 | 73.2 | 26.7 | 0.37 |
| S42-20 | 6.0 | 247 | 8.5 | 708 | 291 | 0.41 |
| S42-21 | 5.5 | 2.46 | 8.0 | 69.0 | 30.8 | 0.45 |
| S42-22 | 5.5 | 2.45 | 79 | 69.0 | 309 | 0.45 |
| S42-23 | 6.7 | 2.45 | 9.2 | 73.3 | 266 | 0.36 |
| S42-24 | 7.2 | 2.42 | 96 | 746 | 252 | 0.34 |
| S42-25 | 5.5 | 2.40 | 8.0 | 69.7 | 302 | 0.43 |
| S42-26 | 5.3 | 230 | 7.6 | 69.7 | 302 | 0.43 |
| S42-27 | 5.1 | 2.28 | 7.4 | 69.2 | 30.8 | 0.44 |
| S42-28 | 6.7 | 2.00 | 87 | 76.9 | 230 | 0.30 |
| S42-29 | 6.0 | 174 | 78 | 775 | 224 | 0.29 |
| S42-30 | 2.2 | 0.49 | 8.5 | 26.3 | 58 | 0.22 |
| S42-31 | 1.9 | 038 | 75 | 24.8 | 50 | 0.20 |
| S42-32 | 1.3 | 0.37 | 7.1 | 18.7 | 52 | 0.28 |
| S42-33 | 1.3 | 035 | 53 | 23.7 | 66 | 0.29 |
| S42-34 | 3.0 | 0.32 | 94 | 31.5 | 34 | 0.11 |
| S42-35 | 1.4 | 032 | 64 | 223 | 50 | 0.22 |
| S42-36 | 1.6 | 031 | 68 | 23.3 | 45 | 0.19 |
| S42-37 | 21 | 0.30 | 76 | 28.2 | 39 | 0.14 |
| S42-38 | 1.9 | 0.28 | 6.7 | 29.2 | 42 | 0.14 |
| S42-39 | 1.5 | 0.27 | 69 | 21.9 | 39 | 0.16 |
| S42-40 | 2.1 | 027 | 7.2 | 29.6 | 37 | 0.13 |
| S42-41 | 1.9 | 027 | 102 | 184 | 26 | 0.14 |
| S42-42 | 1.7 | 0.26 | 7.3 | 22.7 | 36 | 016 |
| S42-43 | 2.1 | 026 | 90 | 23.0 | 29 | 012 |
| S42-44 | 2.0 | 026 | 9.8 | 20.1 | 2.6 | 0.13 |
| S42-45 | 1.8 | 0.25 | 64 | 28.2 | 39 | 0.14 |
| S42-46 | 2.0 | 0,25 | 81 | 24.8 | 31 | 0.12 |
| S42-47 | 1.7 | 0.24 | 7.2 | 23.5 | 34 | 0.14 |
| S42-48 | 2.2 | 0.24 | 74 | 29.5 | 32 | 0.11 |
| S42-49 | 2.1 | 0.24 | 8.0 | 27.0 | 30 | 0.11 |
| S42-50 | 2.3 | 021 | 82 | 27.7 | 26 | 0.09 |
| S42-51 | 1.1 | 021 | 5.9 | 18.6 | 3.5 | 0.19 |
| S42-52 | 2.0 | 0.20 | 6.3 | 31.6 | 32 | 0.10 |
| S42-53 | 2.1 | 020 | 7.4 | 27.9 | 2.7 | 010 |
| S42-54 | 1.7 | 020 | 81 | 21.1 | 25 | 0.12 |
| S42-55 | 20 | 020 | 73 | 27.3 | 2.7 | 0.10 |
| S42-56 | 1.6 | 0.19 | 7.4 | 21.2 | 2.6 | 0.12 |
| S42-57 | 1.3 | 0.19 | 5.3 | 24.4 | 3.5 | 0.15 |
| S42-58 | 1.9 | 0.18 | 5.7 | 32.6 | 3.2 | 0.10 |
| S42-59 | 2.1 | 018 | 75 | 27.9 | 2.4 | 0.09 |
| S42-60 | 1.7 | 0.17 | 71 | 23.7 | 2.5 | 0.10 |
| S42-61 | 2.0 | 0.17 | 7.4 | 26.8 | 2.3 | 0.09 |
| S42-62 | 1.0 | 017 | 4.6 | 22.9 | 3.7 | 0.16 |
| S42-63 | 1.8 | 0.16 | 7.1 | 25.1 | 2.3 | 0.09 |
| S42-64 | 1.8 | 016 | 7.3 | 24.0 | 2.2 | 0.09 |
| S42-65 | 22 | 016 | 86 | 260 | is | 007 |
| S42-66 | 1.7 | 0.15 | 7.1 | 23.6 | 2.1 | 0.09 |
| S42-67 | 1.6 | 0.15 | 68 | 23.4 | 2.2 | 0.09 |
| S42-68 | 1.9 | 0.14 | 7.5 | 24.7 | 1.9 | 0.06 |
| S42-69 | 1.9 | 0.14 | 6.4 | 29.4 | 2.2 | 0.08 |
| S42-70 | 1.4 | 0.14 | 6.7 | 20.7 | 2.1 | 010 |
| S42-71 | 2.1 | 014 | B.8 | 23.9 | 1.6 | 0.07 |
| S42-72 | 1 5 | 013 | 69 | 21.8 | 1.9 | 0.09 |
| S42-73 | 1.3 | 0.12 | 6.3 | 20.5 | 1.9 | 0.09 |
| S42-74 | 08 | 0.11 | 44 | 178 | 2.6 | 0.14 |
| S42-75 | 1.4 | 0.11 | 61 | 23.5 | 1.8 | 0.08 |
| S42-76 | 12 | 0.10 | 5,8 | 19.9 | 17 | 0.09 |
| S42-77 | 1.1 | 0.10 | 62 | 18.1 | 15 | 0.09 |
| S42-78 | 1,3 | 0.09 | 6.0 | 20.9 | 1.5 | 0.07 |
| S42-79 | 06 | 0.06 | 63 | 92 | 1.0 | 0.11 |
| S42-80 | 0.9 | 0.05 | 6.7 | 13.1 | 0.8 | 0.00 |
| S42-81 | 09 | 0.05 | 8.3 | 103 | 0.6 | 0.05 |
| S42-82 | 0.9 | 0.04 | 7.1 | 13.2 | 0.6 | 0.05 |
| S42-83 | 0.9 | 0.04 | 60 | 14.7 | 0.7 | 0.05 |
| S42-84 | 04 | 0.04 | 50 | 8.5 | 08 | 0.09 |
| S42-85 | 0.9 | 0.03 | 90 | 9.4 | 0.3 | 0.04 |
| S42-86 | 06 | 0.03 | 65 | 100 | 05 | 005 |
| S42-87 | 0.5 | 003 | 6.1 | 7.5 | 0.5 | 0.06 |
| S42-88 | 07 | 003 | 5.8 | 11.7 | 0.5 | 0.04 |
| S42-89 | 0.4 | 0.03 | 50 | 7.7 | 0.5 | 0.07 |
| S42-90 | 0.7 | 0.03 | 66 | 10.8 | 0.4 | 0.04 |
| S42-91 | 07 | 0.03 | 60 | 11.4 | 04 | 0.04 |
| S42-92 | 0.7 | 002 | 7.3 | 10.0 | 0.3 | 0.03 |
| S42-93 | 04 | 0.02 | 56 | 6.7 | 03 | 0.05 |
| S42-94 | 0.4 | 002 | 5.5 | 7.9 | 0.3 | 0.04 |
| S42-95 | 05 | 002 | 48 | 96 | 0.3 | 0.03 |
| S42-96 | 06 | 0.02 | 62 | 9.3 | 0.2 | 0.03 |

As shown in the results, S42-1 to S42-78 in Population B contained RebE in an amount exceeding 0.06% by mass based on a dried leaf, i.e., were rich in RebE. Among them, S42-1 to S42-29 contained RebE in an amount exceeding 0.49% by mass based on a dried leaf, i.e., were particularly rich in RebE.

### Example 2: Detection of genetic feature unique to stevia plant with high RebE content

Genomic DNA was extracted from the fresh leaves of some individuals tested in Example 1, and analyzed for genetic features using a sequencer (HiSeq 2500, Illumina, Inc.). As a result, genetic features (A) to (E) tended to be detected in the lines with high RebE content. Then, in order to efficiently detect the genetic features, dCAPS primers for detecting the genetic feature (A) were generated, and the remaining individuals were evaluated for the presence or absence of these genetic features by the dCAPS method. The study by the dCAPS method was omitted for the genetic features (B) to (E), because their variation sites were positioned close to that of the genetic feature (A) on the genome and were likely to be in linkage disequilibrium.

The following dCAPS primers and restriction enzymes were used.

**Table 8 Sequence of dCAPS primers and restriction enzyme**

| Genetic feature | Forward primer | Reverse primer | Restriction enzyme |
|---|---|---|---|
| (A) | AATTATATGATAGAAATTG TTTTCACATTTTGTGTGC CG (SEQ ID NO: 36) | TGGACTAGCGCATATAGG TTAAAGTA (SEQ ID NO: 37) | RsaI |

The detection of the genetic feature by the dCAPS method was performed as follows. First, genomic DNA was extracted from the fresh leaves of each individual tested in Example 1, and PCR was performed using the above dCAPS primers for each genetic feature. The above restriction enzyme was added to the PCR product, and enzymatic reaction was performed at 37° C. The restriction enzyme-treated products were electrophoresed using a microchip type electrophoresis apparatus LabChip GX Touch HT (PerkinElmer, Inc.). The presence or absence of the genetic feature was determined on the basis of the obtained band pattern. Specifically, an individual for which only a band of a non-degraded product was found was determined as A'; an individual for which bands of both a degradation product and a non-degradation product were found was determined as A"; and an individual for which only a band of a degradation product was found was determined as ×. A' and A" were evaluated as having the genetic feature (A), and × was evaluated as not having the genetic feature (A). Particularly, A' was evaluated as having the genetic feature (A').

As shown in the table below, in both Populations A and B, stevia plants with high RebE content tended to have the genetic feature (A). On the other hand, the stevia plants with low RebE content (0.10% by mass or less for Population A; 0.06% by mass or less for Population B) tended to not have the genetic feature (A). Further, the stevia plants with particularly high RebE content (RebE content of 2.10% or more in S41-1 to S41-22, RebE content of 1.74% or more in S42-1 to S42-29) had the genetic feature (A').

**Table 9 Genetic feature in Population A**

| **Line number** | **Allele relating to genetic feature (A)** |
|---|---|
| S41-1 | A' |
| S41-2 | A' |
| S41-3 | A' |
| S41-4 | A' |
| S41-5 | A' |
| S41-6 | A' |
| S41-7 | A' |
| S41-8 | A' |
| S41-9 | A' |
| S41-10 | A' |
| S41-11 | A' |
| S41-12 | A' |
| S41-13 | A' |
| S41-14 | A' |
| S41-15 | A' |
| S41-16 | A' |
| S41-17 | A' |
| S41-18 | A' |
| S41-19 | A' |
| S41-20 | A' |
| S41-21 | A' |
| S41-22 | A' |
| S41-23 | A" |
| S41-24 | A" |
| S41-25 | A" |
| S41-26 | A" |
| S41-27 | A" |
| S41-28 | A" |
| S41-29 | A" |
| S41-30 | A" |
| S41-31 | A" |
| S41-32 | A" |
| S41-33 | A" |
| S41-34 | A" |
| S41-35 | A" |
| S41-96 | A" |
| S41-37 | A" |
| S41-38 | A" |
| S41-39 | A" |
| S41-40 | A" |
| S41-41 | A" |
| S41-42 | A" |
| S41-43 | A" |
| S41-44 | A" |
| S41-45 | A" |
| S41-46 | A" |
| S41-47 | A" |
| S41-48 | A" |
| S41-49 | A" |
| S41-50 | A" |
| S41-51 | A" |
| S41-52 | A" |
| S41-53 | A" |
| S41-54 | A" |
| S41-56 | A" |
| S41-56 | A" |
| S41-57 | A" |
| S41-58 | A" |
| S41-59 | A" |
| S41-60 | A" |
| S41-61 | A" |
| S41-62 | A" |
| S41-63 | A" |
| S41-64 | A" |
| S41-65 | A" |
| S41-66 | A" |
| S41-67 | A' |
| 541-68 | A" |
| S41-69 | A" |
| S41-70 | A" |
| S41-71 | A" |
| S41-72 | A" |
| S41-73 | A" |
| S41-74 | × |
| S41-75 | × |
| S41-76 | × |
| S41-77 | × |
| S41-78 | × |
| S41-79 | × |
| S41-80 | × |
| S41-81 | × |
| S41-82 | × |
| S41-83 | × |
| S41-84 | × |
| S41-85 | × |
| S41-86 | × |
| S41-87 | × |
| S41-88 | × |
| S41-89 | A" |
| S41-90 | × |
| S41-91 | × |
| S41-92 | × |
| S41-93 | × |
| S41-94 | × |
| S41-95 | × |
| S41-96 | × |

**Table 10 Genetic feature in Population B**

| **Line number** | **Allele relating to genetic feature (A)** |
|---|---|
| S42-1 | A' |
| S42-2 | A' |
| S42-3 | A' |
| S42-4 | A' |
| S42-5 | A' |
| S42-6 | A' |
| S42-7 | A' |
| S42-8 | A' |
| S42-9 | A' |
| S42-10 | A' |
| S42-11 | A' |
| S42-12 | A' |
| S42-13 | A' |
| S42-14 | A' |
| S42-15 | A' |
| S42-16 | A' |
| S42-17 | A' |
| 542-18 | A' |
| S42-19 | A' |
| S42-20 | A' |
| S42-21 | A' |
| S42-22 | A' |
| S42-23 | A' |
| S42-24 | A' |
| S42-26 | A' |
| S42-26 | A' |
| S42-27 | A' |
| S42-28 | A' |
| S42-29 | A' |
| S42-30 | A" |
| S42-31 | A" |
| S42-32 | A" |
| S42-33 | A" |
| S42-34 | A" |
| S42-35 | A" |
| S42-36 | A" |
| S42-37 | A" |
| S42-38 | A" |
| S42-39 | A" |
| S42-40 | A" |
| S42-41 | A" |
| S42-42 | A" |
| S42-43 | A" |
| S42-44 | A" |
| S42-45 | A" |
| S42-46 | A" |
| S42-47 | A" |
| S42-48 | A" |
| S42-49 | A" |
| S42-50 | A" |
| S42-51 | A" |
| S42-52 | A" |
| S42-53 | A" |
| S42-54 | A" |
| S42-5.5 | A" |
| S42-56 | A" |
| S42-57 | A" |
| S42-68 | A" |
| S42-59 | A" |
| S42-60 | A" |
| S42-61 | A" |
| S42-62 | A" |
| S42-63 | A" |
| S42-64 | A" |
| S42-65 | A" |
| S42-66 | A" |
| S42-67 | A" |
| S42-68 | A" |
| S42-69 | A" |
| S42-70 | A" |
| S42-71 | A" |
| S42-72 | A" |
| S42-73 | A" |
| S42-74 | A" |
| S42-75 | A" |
| S42-76 | A" |
| S42-77 | A" |
| S42-78 | A" |
| S42-79 | × |
| S42-80 | × |
| S42-81 | × |
| S42-82 | × |
| S42-83 | × |
| S42-84 | × |
| S42-85 | × |
| S42-86 | × |
| S42-87 | × |
| S42-88 | × |
| S42-89 | × |
| S42-90 | × |
| S42-91 | × |
| S42-92 | × |
| S42-93 | × |
| S42-94 | × |
| S42-95 | × |
| S42-96 | × |

As is evident from the above results, the lines having the genetic feature(s) of the present invention tended to have high RebE content (approximately 1.13% on average and 4.16 % at the maximum for Population A; approximately 1.17% on average and 3.52% at the maximum for Population B). The lines having the genetic feature (A') of the present invention had particularly high RebE content (approximately 2.98% on average and 4.16% at the maximum for Population A; approximately 2.79% on average and 3.52 % at the maximum for Population B).

The lines having the genetic feature(s) of the present invention tended to have a high mass ratio of RebE to TSG (2.4% at the minimum and 45.8% at the maximum for Population A; 1.5% at the minimum and 39.3% at the maximum for Population B). The lines having the genetic feature (A') of the present invention tended to have a particularly high mass ratio of RebE to TSG (25.5% at the minimum and 45.8% at the maximum for Population A; 22.4% at the minimum and 39.3% at the maximum for Population B).

The RebE content with respect to stevioside was 1 or less in the lines having the genetic feature(s) of the present invention (0.11 to 0.85 for Population A; 0.07 to 0.65 for Population B). RebE content with respect to stevioside was 0.07 to 0.85 in the lines having the genetic feature (A') of the present invention (0.35 to 0.85 for Population A; 0.29 to 0.65 for Population B).

### INDUSTRIAL APPLICABILITY

The present invention enables the more efficient provision of useful steviol glycoside such as RebE and can therefore promote the provision of a food or beverage, a sweetener composition, a flavor or a medicament, etc. comprising such steviol glycoside and thereby having good quality of taste.

## Claims

1. A stevia plant having at least one of the following genetic features (A) to (E).
(A) Homozygous or heterozygous for the allele wherein the base at the position corresponding to position 40 of SEQ ID NO: 1 is C.
(B) Homozygous or heterozygous for the allele wherein the base at the position corresponding to position 21 of SEQ ID NO: 2 is T.
(C) Homozygous or heterozygous for the allele wherein the base at the position corresponding to position 28 of SEQ ID NO: 3 is T.
(D) Homozygous or heterozygous for the allele wherein the base at the position corresponding to position 59 of SEQ ID NO: 4 is C.
(E) Homozygous or heterozygous for the allele wherein the base at the position corresponding to position 64 of SEQ ID NO: 5 is T.

2. The plant according to claim 1, comprising 0.07 % or more of rebaudioside E per unit mass of a dried leaf.

3. The plant according to claim 1 or 2, wherein the plant is a non-genetically modified plant.

4. The plant according to any one of claims 1 to 3, wherein the plant includes a stevia plant subjected to a mutagenesis treatment and a progeny plant thereof.

5. A seed, a dried leaf, a tissue, a tissue culture or a cell of the plant according to any one of claims 1 to 4.

6. The tissue, tissue culture or cell according to claim 6, which is selected from an embryo, a meristem cell, a pollen, a leaf, a root, a root apex, a petal, a protoplast, a leaf section and a callus.

7. A method of producing a high rebaudioside E-content stevia plant comprising 0.07 % or more of rebaudioside E per unit mass of a dried leaf, the method comprising a step of crossing the stevia plant according to any one of claims 1 to 4 with a second stevia plant.

8. The method according to claim 7, wherein the second plant is the stevia plant according to any one of claims 1 to 4.

9. An extract of the plant according to any one of claims 1 to 4, or of the seed, dried leaf, tissue, tissue culture or cell according to claim 5, wherein the extract comprises rebaudioside E.

10. A food or beverage, a sweetener composition, a flavor or a medicament, comprising the extract according to claim 9.

11. A method of producing an extract comprising rebaudioside E, comprising a step of obtaining an extract comprising rebaudioside E from the plant according to any one of claims 1 to 4, or from the seed, dried leaf, tissue, tissue culture or cell according to claim 5.

12. A method of producing rebaudioside E, comprising a step of purifying rebaudioside E from the extract comprising rebaudioside E according to claim 11.

13. A method of producing a food or beverage, a sweetener composition, a flavor or a medicament, comprising:
a step of providing an extract of the plant according to any one of claims 1 to 4, an extract of the seed, dried leaf, tissue, tissue culture or cell according to claim 5, or the extract according to claim 11; and
a step of adding the extract to a raw material for the food or beverage, sweetener composition, flavor or medicament.

14. A method of screening for the stevia plant according to any one of claims 1 to 4, comprising a step of detecting from the genome of a test plant the presence and/or the absence of at least one of the following genetic features (A) to (E).
(A) Homozygous or heterozygous for the allele wherein the base at the position corresponding to position 40 of SEQ ID NO: 1 is C.
(B) Homozygous or heterozygous for the allele wherein the base at the position corresponding to position 21 of SEQ ID NO: 2 is T.
(C) Homozygous or heterozygous for the allele wherein the base at the position corresponding to position 28 of SEQ ID NO: 3 is T.
(D) Homozygous or heterozygous for the allele wherein the base at the position corresponding to position 59 of SEQ ID NO: 4 is C.
(E) Homozygous or heterozygous for the allele wherein the base at the position corresponding to position 64 of SEQ ID NO: 5 is T.

15. The method according to claim 14, further comprising a step of measuring the content of rebaudioside E in a leaf tissue.

16. The method according to claim 14 or 15, wherein the plant obtained by the screening comprises 0.07 % or more of rebaudioside E per unit mass of a dried leaf.
